# EUROPEAN PATENT SPECIFICATION

(11) **EP 3 107 996 B1**
(45) Date of publication and mention of the grant of the patent: **19.02.2020**
(21) Application number: 15751942.2
(22) Date of filing: 20.02.2015
(51) Int. Cl.: C12N 5/10, A61K 35/17, A61K 39/00, G01N 33/50

(54) **TSCM CELLS AND METHODS FOR USE**
TSCM-ZELLEN UND VERFAHREN ZUR VERWENDUNG
CELLULES TSCM ET LEURS PROCÉDÉS D'UTILISATION

(30) Priority: 21.02.2014 US 201461943278 P; 03.03.2014 US 201461947355 P
(43) Date of publication of application: 28.12.2016
(73) Proprietor: Colmmune, Inc., Durham, NC 27704 (US)
(72) Inventor: DEBENEDETTE, Mark, Durham, NC 27704 (US); NICOLETTE, Charles, Durham, NC 27704 (US); HORVATINOVICH, Joseph, Durham, NC 27704 (US)
(74) Representative: HGF Limited
(86) International application number: PCT/US2015/016797
(87) International publication number: WO 2015/127190

(56) References cited:
- US-A1- 2008 311 155
- US-A1- 2009 274 669
- US-A1- 2012 269 832
- US-A1- 2013 280 805
- GATTINONI LUCA ET AL: "A human memory T cell subset with stem cell-like properties", NATURE MEDI, NATURE PUB. CO, vol. 17, no. 10, 1 October 2011 (2011-10-01), pages 1290-1298, XP009168103, ISSN: 1078-8956, DOI: 10.1038/NM.2446
- N. CIERI ET AL: "IL-7 and IL-15 instruct the generation of human memory stem T cells from naive precursors", BLOOD, vol. 121, no. 4, 24 January 2013 (2013-01-24), pages 573-584, XP055386326, US ISSN: 0006-4971, DOI: 10.1182/blood-2012-05-431718
- DEBENEDETTE ET AL.: 'Priming of a novel subset of CD 28+ rapidly expanding high-avidity effector memory CTL by post maturation electroporation- CD 40L dendritic cells is IL -12 dependent' J IMMUNOL. vol. 181, no. 8, 15 October 2008, ISSN 0022-1767 pages 5296 - 5305, XP055162515

## Description

### FIELD OF THE INVENTION

The present invention relates to an *in vitro* method for obtaining an autologous population of cells enriched for T_{SCM} cells suitable for introduction into a human patient. The present disclosure also relates to T_{SCM} cells and uses thereof T_{SCM} cells can be used to help identify and treat patients who are likely to experience particular treatment outcomes.

### BACKGROUND

Cell therapy utilizes modified antigen presenting cells (APCs) or immune effector cells to initiate an immune response in a patient. Antigen presenting cells are central to cell therapy because they initiate the immune response; specifically, they are capable of inducing a primary immune response from T lymphocytes.

Dendritic cells (DC) are the most potent APCs involved in adaptive immunity. They coordinate the initiation of immune responses by naive T cells and B cells and induce antigen-specific cytotoxic T lymphocyte (CTL) responses. DCs are specialized in several ways to prime helper and killer T cells *in vivo.* For example, immature DCs that reside in peripheral tissues are equipped to capture antigens and to produce immunogenic MHC-peptide complexes. In response to maturation-inducing stimuli such as inflammatory cytokines, immature DCs develop into potent T cell stimulators by upregulating adhesion and costimulatory molecules and migrate into secondary lymphoid organs to select and stimulate rare antigen-specific T cells. However, potent stimulation of T cells occurs only after DC maturation, a process that increases the availability of MHC/peptide complexes on the cell surface in addition to co-stimulatory molecules that direct the effector function of the responding T-cells.

Co-stimulation is typically necessary for a T cell to produce sufficient cytokine levels that induce clonal expansion. One characteristic of dendritic cells that makes them potent antigen presenting cells is that they are rich in co-stimulatory molecules of the immune response, such as the molecules CD80 and CD86, which activate the molecule CD28 on T lymphocytes. In return, T-helper cells express CD40L, which ligates CD40 on DCs. These mutual interactions between DCs and T cells leads to 'maturation' of the DCs and the development of effector function in the T cells. The expression of adhesion molecules, like the molecule CD54 or the molecule CD11a/CD18, facilitate the co-operation between the DCs and the T cells. Another special characteristic of DCs is to deploy different functions depending on their stage of differentiation. Thus, the capture of antigen and its transformation are the two principal functions of the immature dendritic cell, whereas its capacities to present the antigen in order to stimulate the T cells increases as the DCs migrate into the tissues and the lymphatic ganglia. This change of functionality corresponds to a maturation of the dendritic cell. Thus, the passage of the immature dendritic cell to the mature dendritic cell represents a fundamental step in the initiation of the immune response. Traditionally, this maturation was followed by monitoring the change of the surface markers on the DCs during this process.

Some of the more important cell surface markers characteristic of the different stages of maturation of the dendritic cells include CD34+ for hematopoietic stem cell; CD14++, DR+, CD86+, CD16+/-, CD54+, and CD40+for monocytes; CD14+/-, CD16-, CD80+/-, CD83-, CD86+, CD1a+, CD54+, DQ+, DR++ for immature dendritic cells, and CD14-, CD83++, CD86++, CD80++, DR+++, DQ++, CD40++, CD54++, CD1a +/- for mature dendritic cells, where "+" indicates positive expression, "++" indicates higher expression, "+/-" indicates weaker expression, and "-" indicates very weak or undetectable expression. However, the surface markers can vary depending upon the maturation process.

Mature DCs are currently preferred to immature DCs for immunotherapy. Only fully mature DC progeny lack GM-CSF Receptor (GM-CSF-R) and remain stably mature upon removal and/or in the absence of GM-CSF. Also, mature DCs have been shown to be superior in inducing T cell responses *in vitro* and *in vivo.* Mature dendritic cells also are useful to take up and present antigen to T-lymphocytes *in vitro* or *in vivo.* The modified, antigen presenting DCs and/or T cells educated from these modified DCs have many applications, including diagnostic, therapy, vaccination, research, screening and gene delivery.

It is difficult to isolate mature dendritic cells from peripheral blood because less than 1% of the white blood cells belongs to this category. Mature DCs are also difficult to extract from tissues. This difficulty, in combination with the potential therapeutic benefit of DCs in cell therapy, has driven research and development toward new methods to generate mature dendritic cells using alternative sources. Several methods are reported to produce mature DCs from immature dendritic cells, and it has been shown that different methods can produce mature DCs with different properties. Methods that produce mature DCs with particularly advantageous properties include those disclosed in WO2006042177 (Healey et al.); WO2007117682 (Tcherepanova et al.); DeBenedette et al. (2008) J. Immunol. 181: 5296-5305; and Calderhead et al. (2008) J. Immunother. 31: 731-41. These methods in particular produce mature DCs designated "PME-CD40L" DCs, discussed further herein below.

The generation of T_{SCM} cells comprising activating T-lymphocytes with anti-CD3/CD28-conjugated magnetic beads and then culturing in the presence of IL-1 and IL-15 has been described in Gattinoni, et al., 2011. Gattinoni, et al. 2010 also describes that infusion of T_{SCM} cells in immunodeficient mice induces an immune response. The generation of human T_{SCM} cells by activating T-cells with α-CD3/CD28 beads in the presence of the GSK-3β inhibitor TWS119 is described in Cieri et al., 2013. US 2013/280805 describes a method for producing PME-CD40L mature DCs.

### SUMMARY OF THE INVENTION

Previously, it was discovered that PME-CD40L DCs can be used to treat a human patient having an immune disease or disorder and also to stimulate the production *in vivo* of advantageous T cells. PME-CD40L DCs are produced by methods previously developed and described in WO2006042177 (Healey et al.); WO2007117682 (Tcherepanova et al.); DeBenedette et al. (2008) J. Immunol. 181: 5296-5305; and Calderhead et al. (2008) J. Immunother. 31: 731-41. PME-CD40L DCs can be produced, for example, by a method comprising the sequential steps of: (a) culturing isolated immature dendritic cells (iDCs) with an interferon gamma receptor (IFN-γR) agonist in the presence of a TNF-αR agonist and PGE₂ for approximately 12 to 30 hours to produce CD83⁺ mature dendritic cells; and (b) transfecting said CD83⁺ mature dendritic cells (mDCs) with a CD40 agonist to produce a transient CD40 signal. In some examples, the CD40 agonist is mRNA encoding a CD40L polypeptide. In some of these examples, the mRNA encodes a CD40L polypeptide consisting of amino acid residues 21-261 of SEQ ID NO:2 of WO2007117682. The mRNA encoding the CD40L polypeptide may be cotransfected with an mRNA encoding an antigen. PME-CD40L DCs are mature DCs that are also phenotypically CD83⁺ and CCR7⁺.

The inventors have surprisingly discovered that PME-CD40L DCs also stimulate the production of a particular type of T cells known as "stem cell memory" cells, also designated "T_{SCM"} cells, both *in vivo* and *in vitro.* T_{SCM} cells are stem cell memory T cells that are multipotent and can also give rise to progeny cells that are themselves T_{SCM} cells, which makes possible the generation of additional T_{SCM} cells. The production of T_{SCM} cells by exposure to PME-CD40L DCs can occur *in vivo* in human patients having immune diseases or disorders, including AIDS or infection with HIV, and can serve as an indicator of a patient's immune response and thus clinical prognosis. PME-CD40L DCs can also induce T_{SCM} cells *in vitro* by coculturing PME-CD40L DCs with lymphocytes. This coculturing of PME-CD40L DCs with lymphocytes results in a cell population enriched for T_{SCM} cells. These cells can then be reintroduced into a patient to help stimulate the immune response of the patient from whom they were derived (*i.e.,* autologous treatment), and can also be used, for example, to treat another patient in adoptive transfer therapy (*i.e.,* heterologous treatment). In some examples, the T_{SCM} cells are further purified from the lymphocyte population or are further enriched prior to use in adoptive transfer therapy.

In one aspect, the present invention relates to an *in vitro* method for obtaining an autologous population of cells enriched for T_{SCM} cells suitable for introduction into a human patient comprising the steps of:
a) preparing PME-CD40L mature DCs from a tissue source isolated from a human patient;
b) culturing PBMCs obtained from said patient *in vitro* with said PME-CD40L mature DCs in a coculture for a time sufficient to induce an increase in the number of T_{SCM} cells in said coculture;
c) enriching said T_{SCM} cells from said coculture.

In an embodiment, the PME-CD40L mature DCs are loaded with an antigen.

In an embodiment, the DCs are loaded with said antigen by transfection with RNA encoding said antigen.

In an embodiment, the RNA is prepared from cells of an HIV-infected patient.

In an embodiment, the antigen is prepared from cancer cells of a cancer patient.

In an embodiment, the T_{SCM} cells are CD8+, CD95+, CD28+, CCR7+, and CD45RA+.

In an embodiment, the T_{SCM} cells are enriched as cells positive for CD27, CD28, and CD45RA.

In an embodiment, the method further comprises combining said T_{SCM} cells with a pharmaceutically acceptable carrier.

### BRIEF DESCRIPTION OF THE FIGURES

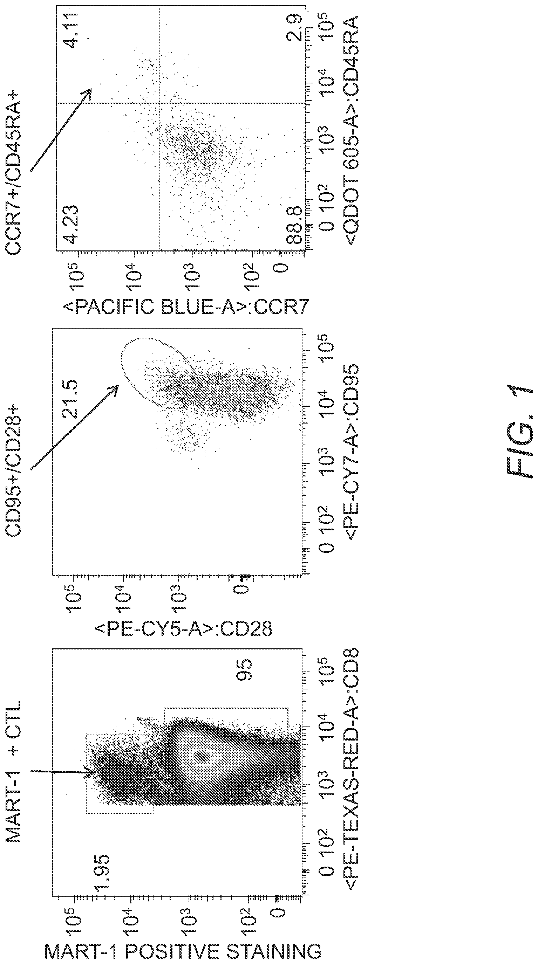

**Figure 1** shows the expansion and detection of MART-1 specific T_{SCM} CTLs. PME-CD40L DCs were used to expand a population of MART-CTLs (see Example 2). Multi-color flow cytometry was used to detect CD8+/CD95+/CD28+/CCR7+/CD45RA+ cells present in co-cultures containing PBMCs and PME-CD40L DCs.

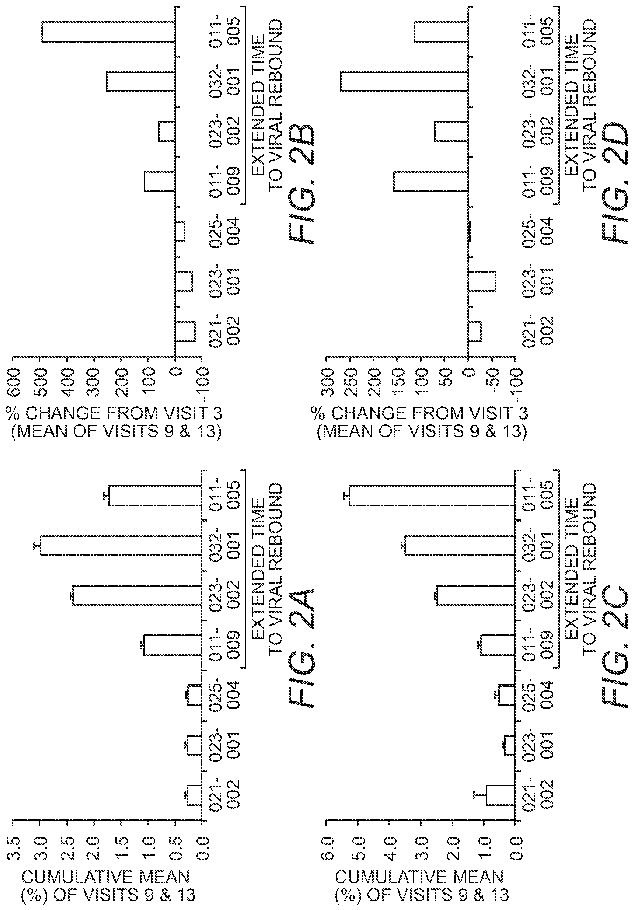

**Figures 2A, 2B, 2C, and 2D** show the percentage of CFSElo CD27+ /CD28+/ CD45RA+ T cells in human clinical patients treated with "AGS-004" (see Example 3). Proliferating T_{SCM} cells expressing CD27, CD28 and CD45RA were identified by gating on the CFSE lo population. Proliferating CD4 T_{SCM} cells (Figure 2A and Figure 2B) and CD8 T_{SCM} cells (Figure 2C and Figure 2D) were further sub-gated to identify the CD27+/CD28+/CD45RA+ population. The cumulative mean of CFSElo T_{SCM} cells was determined at visits 9 and 13 for the CD4 (Figure 2A) and CD8 (Figure 2C) T cell populations by averaging the percentage of CFSElo cells present in the CD27+/CD28+/CD45RA+ gate for both the visit 9 and visit 13 time points. The percent change from visit 3 was determined by dividing the mean average percentage of cells in the CD4 (Figure 2B) or CD8 (Figure 2D) CD27+/CD28+/CD45RA+ T cell gate determined at visit 9 and visit 13 by the percent of cells in the CD4 or CD8 CD27+/CD28+/CD45RA+ T cell determined at visit 3 (baseline).

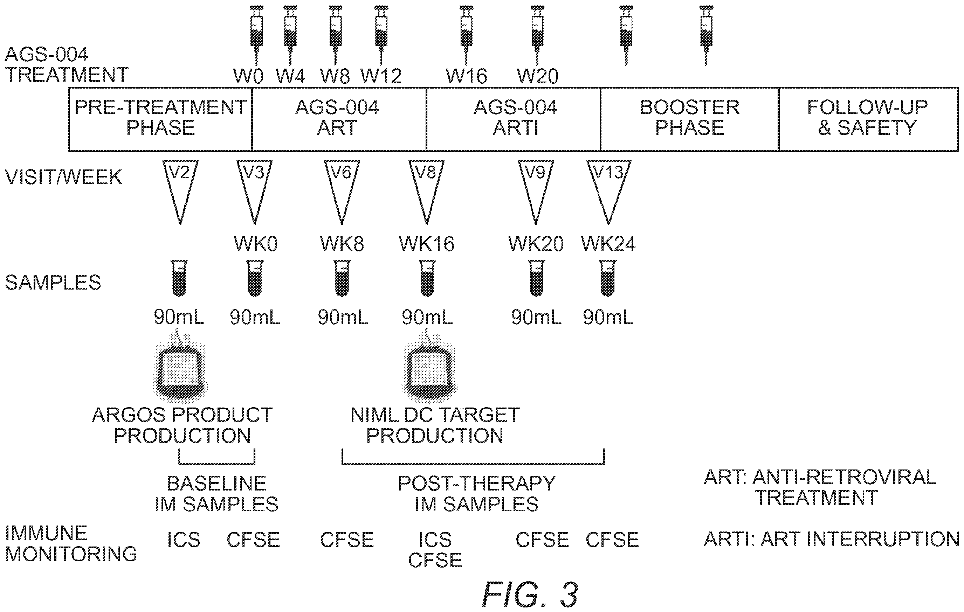

**Figure 3** is a schematic diagram of an Argos Therapeutics' AGS-004 clinical trial in which HIV-infected patients on Anti-Retroviral Therapy (ART) are treated with Argos's AGS-004 treatment, enter Analytical Treatment Interruption (ATI or ARTI, also referred to elsewhere herein as Structured Treatment Interruption or STI), and are monitored for changes in viral load and immune response as indicated.

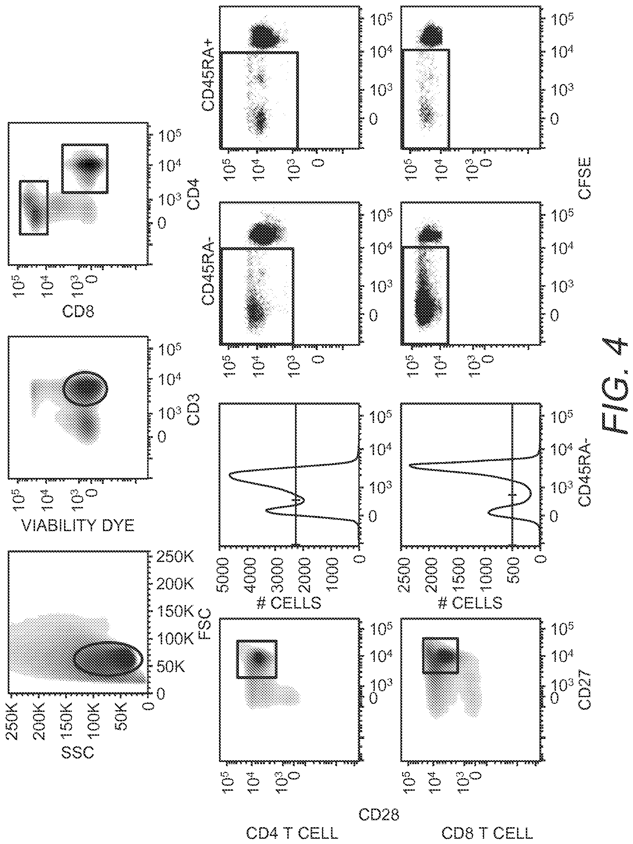

**Figure 4** shows a gating strategy used to identify proliferating T_{SCM} cells (see Example 3).

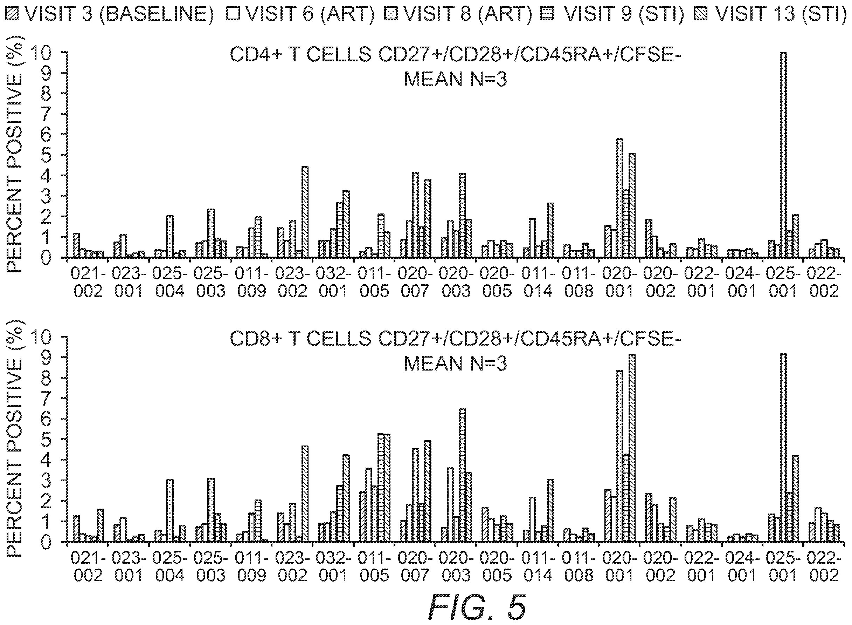

**Figure 5** shows measurements of proliferating CFSElo T cells in patients treated with AGS-004 (see Example 3). Nineteen patients provided complete sets of blood draws for immune analysis. CD4 and CD8 T_{SCM} subsets defined by the expression of CD27, CD28, and CD45RA were identified by gating on the CFSElo population determined at each visit after *in vitro* restimulation with DCs encoding the HIV gene products Gag, Nef, Vpr, and Rev (AGS-004). Patient identifiers are presented on the horizontal axis, and for each patient identifier the values for each visit are shown in the following order from left to right: Visit 3 (baseline); Visit 6 (ART); Visit 8 (ART); Visit 9 (Structured Treatment Interruption ("STI")); and Visit 13 (STI).

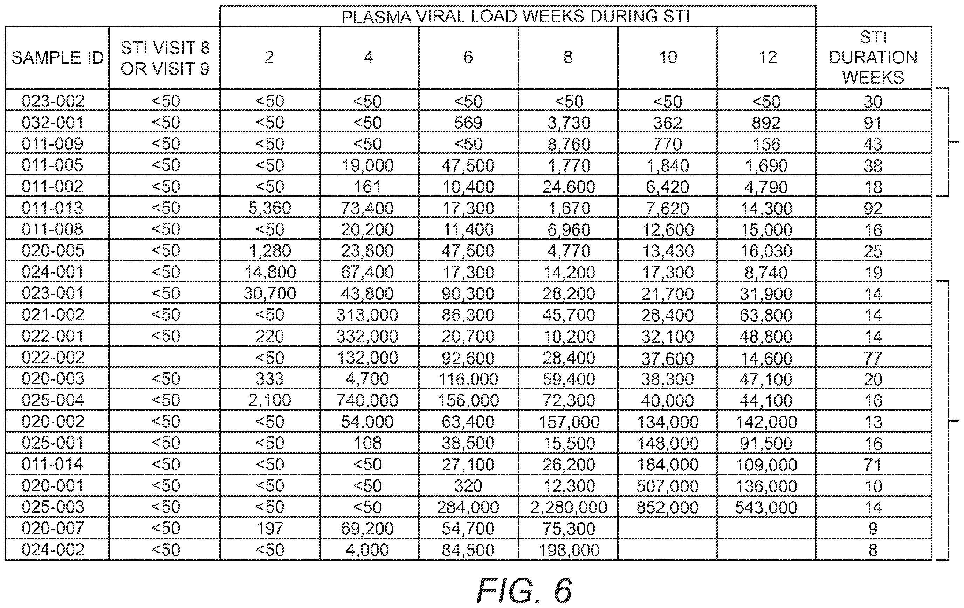

**Figure 6** shows plasma viral load determination at the indicated visits during weeks of STI. Viral load was determined at visit 8 or 9 prior to STI and every 2 weeks during treatment interruption. Values represent number of viral copies/ml detected in plasma at the indicated weeks during STI; values at <50 cells/ml are below the limit of detection. The top bracket indicates patients showing an extended time to viral rebound and the lower bracket indicates patients showing an early time to viral rebound.

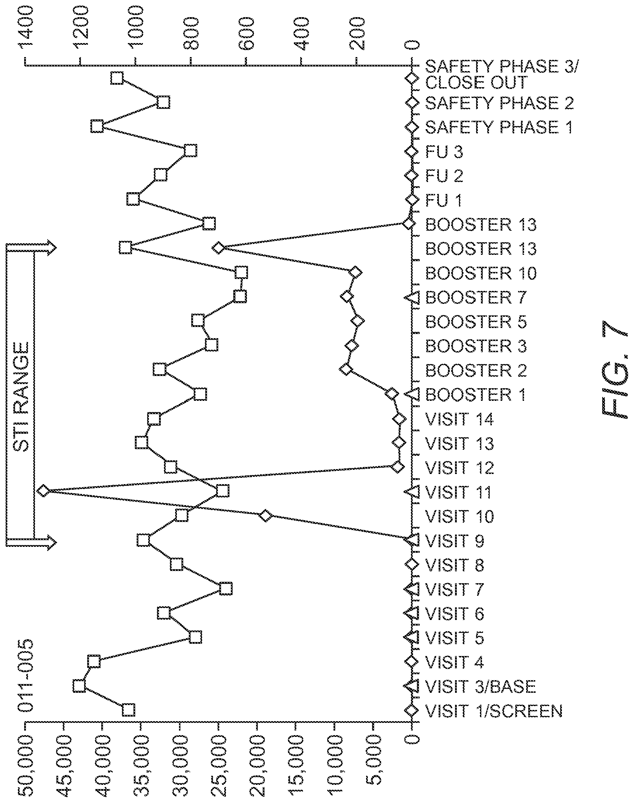

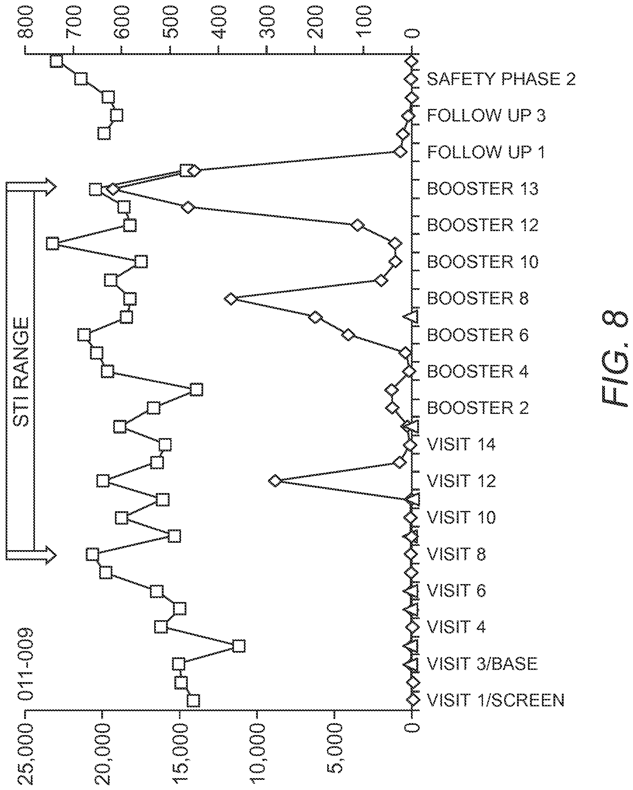

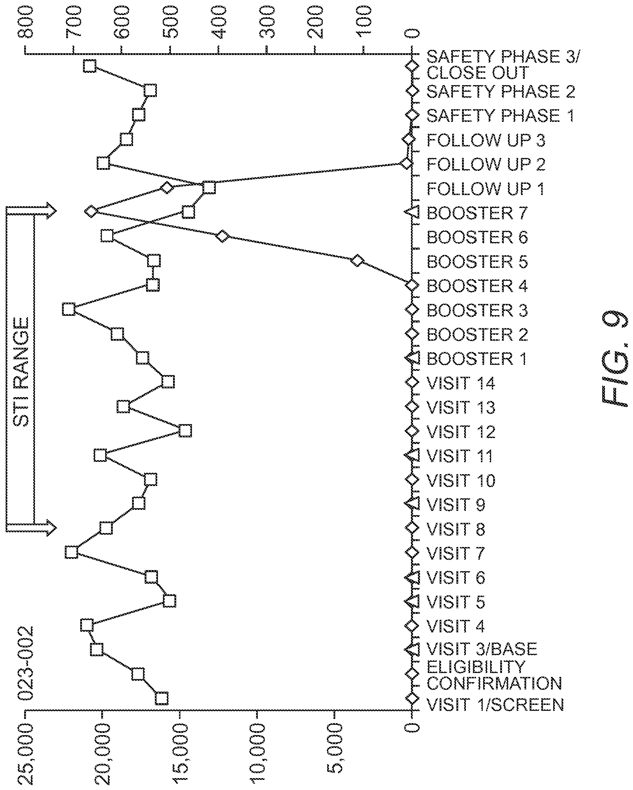

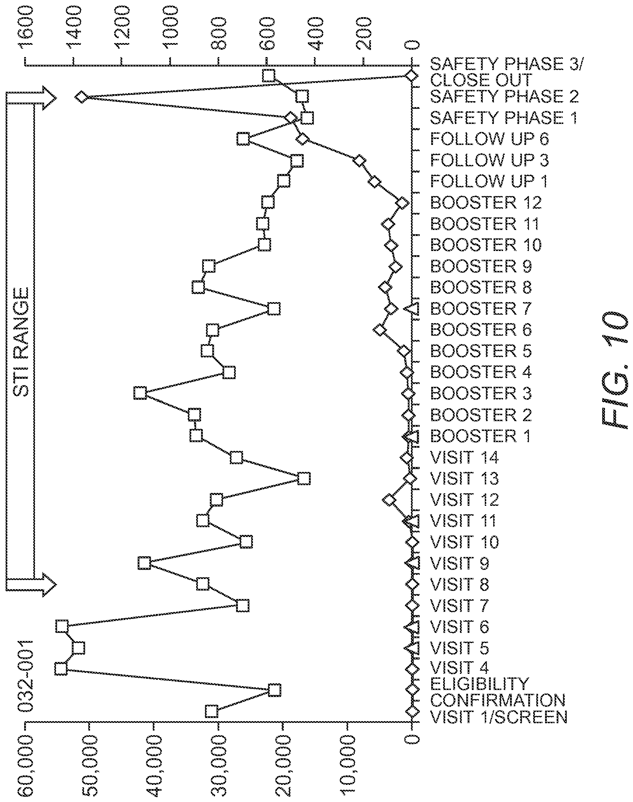

**Figure 7****,** **Figure 8****,** **Figure 9****,** and **Figure 10** show viral load and CD4 T cell counts in subjects with extended time to viral rebound. Viral load values are shown on the left-hand vertical axis and CD4 T cell count values are shown on the right-hand vertical axis. Viral load data points are represented by diamonds, CD4 T cell count data points are represented by squares, and AGS-004 dose visits are represented by triangles (on the horizontal axis); in Figure 7, "Follow Up" visits are abbreviated "FU." Data is shown in Figure 7 for Patient 011-005; in Figure 8 for Patient 011-009; in Figure 9 for Patient 023-002; and in Figure 10 for Patient 032-001.

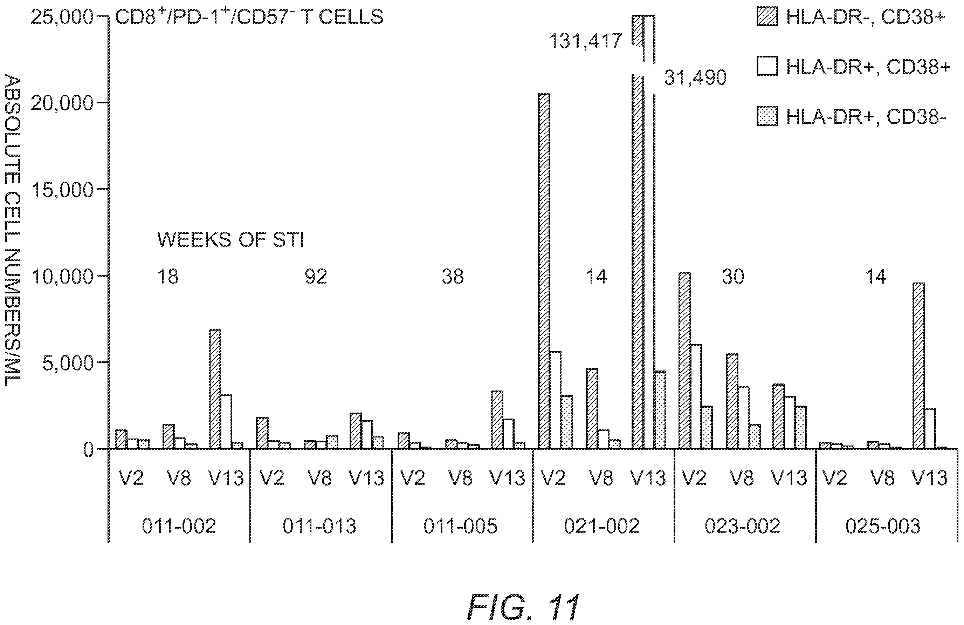

**Figure 11** shows a comparison of PD-1 expression on activated CTL subsets in patients in an AGS-004 clinical trial during STI. The activated phenotype of CD8+ T cells was defined by multi-color flow cytometry of PBMCs collected at Visit 2, Visit 8, and Visit 13 from six patients. The PD-1+/CD57 CD8+ T cell subset was further differentiated by the expression pattern of CD38 and HLA-DR. The number of weeks of STI is indicated above each patient. Counts for cell types shown for each visit are, from left to right, for cells that are also HLA-DR /CD38+; HLA-DR+/CD38+; and HLA-DR+/CD38 .

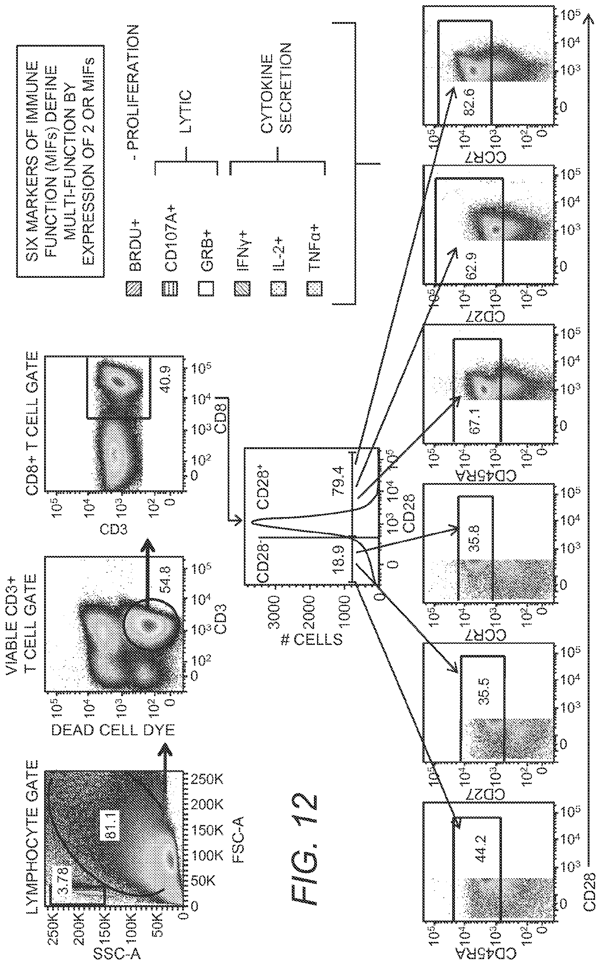

**Figure 12** shows multi-functional T cell responses of PBMCs from experiments described in Example 5.

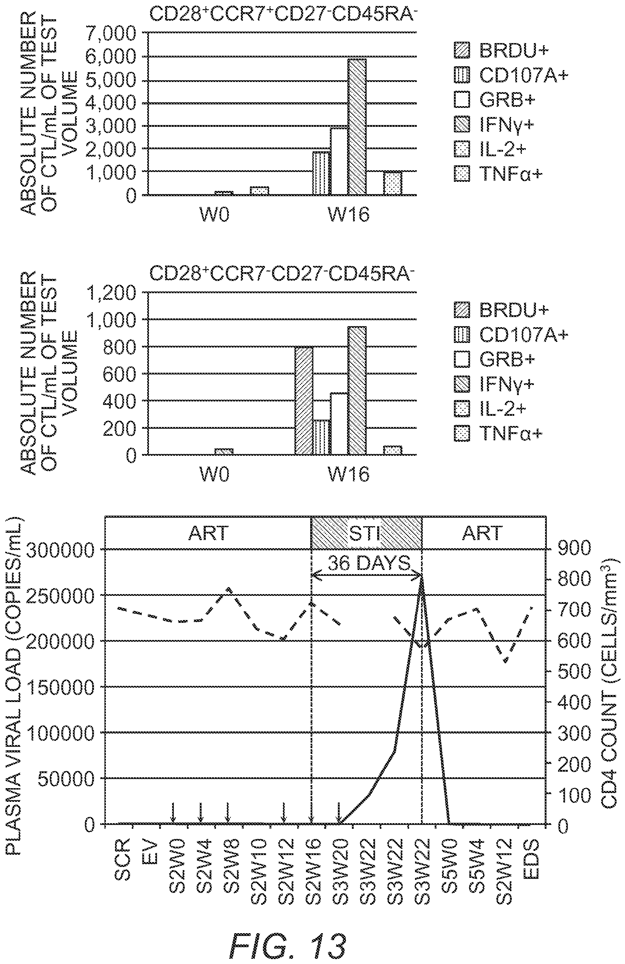

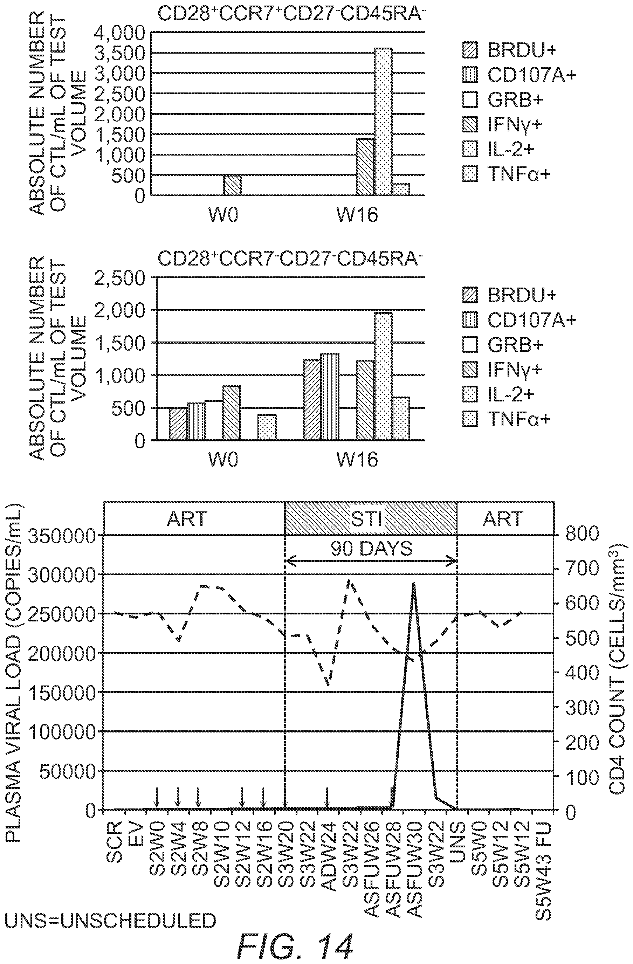

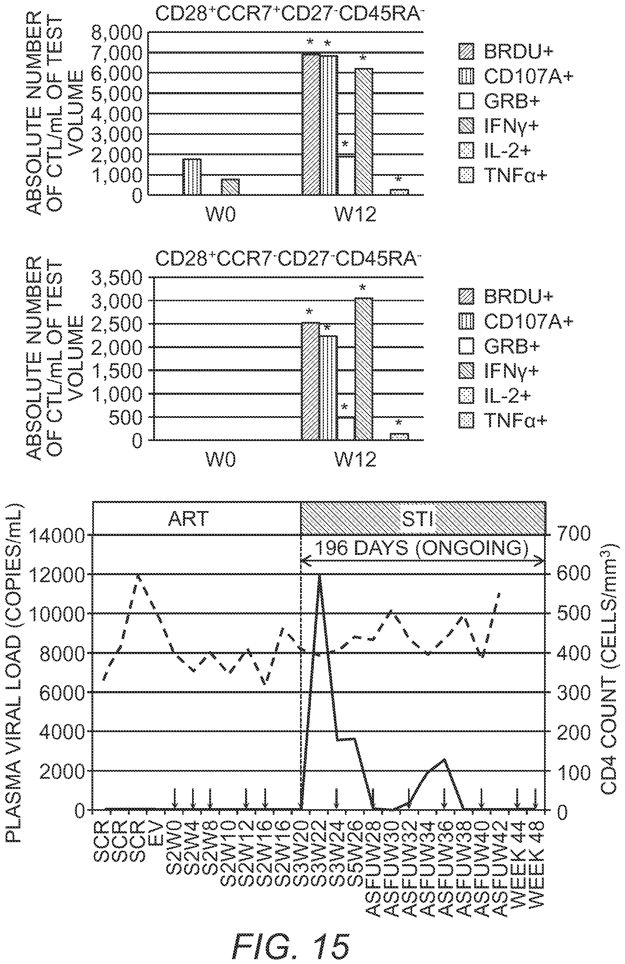

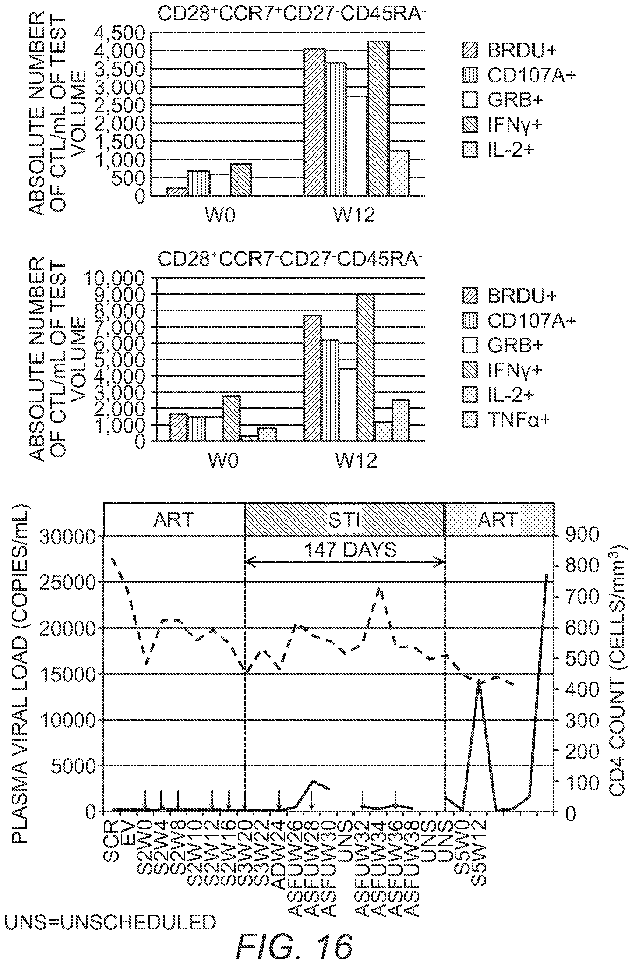

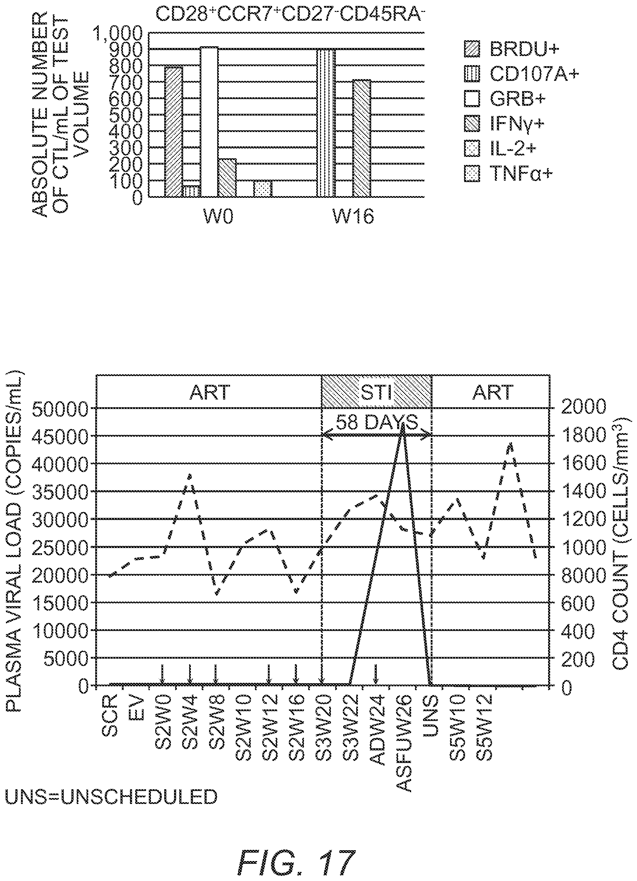

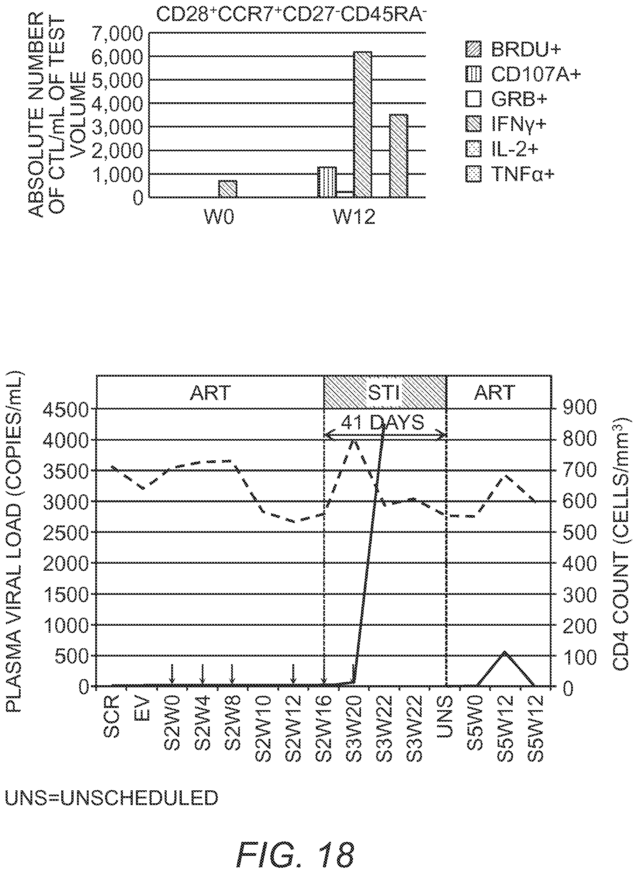

**Figure 13****,** **Figure 14****,** **Figure 15****,** **Figure 16****,** **Figure 17****,** and **Figure 18** show multi-functional immune responses and viral load trajectories before, during, and after ATI with AGS-004 (see Example 5). Absolute numbers of CD28+/CD45RA CTLs for each marker are shown in the bar graphs; for both timepoints, data is shown from left to right for BrdU, CD107a+, Grb+, IFN-gamma+, IL-2+, and TNF-alpha+. Viral load trajectories are shown in the line graph on the right in each figure, with plasma viral load in copies/ml on the left-hand vertical axis and CD4 count in cells/mm³ on the right hand vertical axis. An asterisk above a bar in the bar graph indicates a CTL response that met criteria for positivity that were defined as at least a 2-fold increase in the absolute number of CTLs for a given (test) antigen determined post-dosing in comparison to the number of CTLs for said antigen per-dosing (*i.e.,* at Week 0). Data is shown in Figure 13 for Patient 51-100; in Figure 14 for Patient 54-100; in Figure 15 for Patient 51-102; in Figure 16 for Patient 54-101; in Figure 17 for Patient 54-102; and in Figure 18 for Patient 54-104.

### DETAILED DESCRIPTION OF THE INVENTION

Previously, novel methods were developed to produce mature DCs that are described in detail in: WO2006042177 (Healey et al.); WO2007117682 (Tcherepanova et al.); DeBenedette et al. (2008) J. Immunol. 181: 5296-5305; and Calderhead et al. (2008) J. Immunother. 31: 731-41. In some of these methods, immature DCs are sequentially signaled with a first signal (an IFN-γ receptor agonist and/or a TNF-α receptor agonist) to produce CD83⁺ CCR7⁻ mature DCs and then are signaled with a second signal (a CD40 agonist), producing CD83⁺ CCR7⁺ mature DCs; various IFN-γ receptor agonists and/or TNF-α receptor agonists may be used.

In a method called the "PME-CD40L process" (for Post Maturation Electroporation with CD40L), immature DCs are first phenotypically matured by adding 'inflammatory mediators' IFN-γ and TNF-α to the culture medium; optionally, PGE₂ is also added. Then, approximately 12-30 hours later (in some examples about 18 hrs later), the cells are electroporated with CD40L mRNA and, optionally, antigen-encoding mRNA. This 'PME-CD40L process' produces CD83⁺ CCR7⁺ mature DCs. Cells harvested from this process after electroporation (*e.g.,* 4 hrs post electroporation) and formulated as a vaccine were shown to mediate maximum immunopotency in *in vitro* assays.

Dendritic cells made by the PME-CD40L process (herein, "PME-CD40L DCs") are phenotypically different than previously known dendritic cells. For example, PME-CD40L DCs can support long term antigen-specific CTL effector function and induce a type of effector memory CTLs designated "Rapidly Expanding High-Avidity" ("REHA") cells (see DeBenedette et al. (2008) J. Immunol. 181: 5296-5305). These cells retain the capacity to expand, produce cytokines, and kill target cells--all critical events in mediating robust long-term CTL effector function. Thus, PME-CD40L DCs were shown to preferentially induce a population of CD28⁺ CD45RA memory/effector T cells from a population of antigen-specific T cells, which could be either naive T cells or antigen-experienced T cells. The resulting effector/memory T cells produced IFNγ and IL-2 and could kill target cells; thus, these cells differed from both effector T cells (which produce IFNγ and can kill target cells, but do not produce IL-2) and memory T cells (which produce IFNγ and IL-2, but do not kill target cells).

Surprisingly, it has now been discovered that in addition to inducing "REHA" T cells, PME-CD40L DCs are potent immunostimulatory agents that are useful in producing T_{SCM} cells and thus are useful in methods of the invention. Methods for producing PME-CD40L DCs comprise the sequential steps of: (a) signaling isolated immature dendritic cells (iDCs) with a first signal comprising an interferon gamma receptor (IFN-γR) agonist, and optionally a TNF-αR agonist, to produce IFN-γR agonist signaled dendritic cells; and (b) signaling said IFN-γR agonist signaled dendritic cells with a second transient signal comprising an effective amount of a CD40 agonist to produce CD83⁺ CCR7⁺ mature dendritic cells. In some examples, the CD83⁺ CCR7⁺ mature DCs transiently express CD40L polypeptide; in some instances, the CD40L is predominantly localized intracellularly rather than on the cell surface. At least 60%, at least 70%, at least 80% or at least 90% of the CD40L polypeptide may be localized intracellularly.

PME-CD40L DCs exhibit some distinctive characteristics, including: (i) they demonstrate elevated cell surface expression of co-stimulator molecules CD80, CD83, and CD86; ii) they are CCR7⁺; and iii) they secrete IL-12 p70 polypeptide or protein, and/or secrete significantly reduced levels (0 to 500 pg per ml per million DCs) of IL-10 (see, *e.g.,* data and experiments presented in WO2006042177 (Healey et al.) and WO2007117682 (Tcherepanova et al.)). These mature CD83⁺ CCR7⁺ DCs produce at least 1000 pg IL-12 per 10⁶ DCs; IL-10 and IL-12 levels can be determined by ELISA of culture supernatants collected at up to 36 hrs post induction of DC maturation from immature DCs (Wierda et al. (2000) Blood 96: 2917; Ajdary et al. (2000) Infection and Immunity 68: 1760). One of skill in the art can also determine when PME-CD40Ls have been produced by sampling a cell or small (sub)population of DCs from a cell population for the presence of mature DCs expressing CD40L mRNA and/or CD40L polypeptide, or expressing interleukin 12 (IL-12) p35 protein. Other characteristics of these cells are discussed, for example, in WO2006042177 (Healey et al.); WO2007117682 (Tcherepanova et al.); DeBenedette et al. ((2008) J. Immunol. 181: 5296-5305); and Calderhead et al. ((2008) J. Immunother. 31: 731-41).

Immature DCs used to produce PME-CD40L DCs can be isolated or prepared from a suitable tissue source containing DC precursor cells and differentiated *in vitro* to produce immature DCs. For example, a suitable tissue source can be one or more of: bone marrow cells; peripheral blood progenitor cells (PBPCs); peripheral blood stem cells (PBSCs); and cord blood cells. Preferably, the tissue source is a peripheral blood mononuclear cell (PBMC). The tissue source can be fresh or frozen, and can be pre-treated with an effective amount of a growth factor that promotes growth and differentiation of non-stem or progenitor cells, which are then more easily separated from the cells of interest. These methods are known in the art and described briefly, for example, in Romani et al. ((1994) J. Exp. Med. 180: 83) and Caux et al. ((1996) J. Exp. Med. 184: 695). The immature DCs can also be isolated from peripheral blood mononuclear cells (PBMCs) which optionally are treated with an effective amount of granulocyte macrophage colony stimulating factor (GM-CSF) in the presence or absence of interleukin 4 (IL-4) and/or IL-13, so that the PBMCs differentiate into immature DCs. In some examples, PBMCs are cultured in the presence of GM-CSF and IL-4 for about 4-7 days, preferably about 5-6 days, to produce immature DCs. In some examples, the first signal is given at day 4, 5, 6, or 7, and most preferably at day 5 or 6. In addition, GM-CSF as well as IL-4 and/or IL-13 may be present in the medium at the time of the first and/or second signaling.

To increase the number of dendritic precursor cells in animals, including humans, one can pre-treat subjects with substances which stimulate hematopoiesis. Such substances include but are not limited to G-CSF and GM-CSF. The amount of hematopoietic factor to be administered may be determined by one skilled in the art by monitoring the frequency of cell types in individuals to whom the factor is being administered. U.S. Patent No. 6,475,483 teaches that dosages of G-CSF of 300 micrograms daily for 5 to 13 days and dosages of GM-CSF of 400 micrograms daily for 4 to 19 days result in significant yields of dendritic cells.

Alternatively, the immature dendritic cells can be signaled with an effective amount of a TNF-α receptor agonist followed by signaling with a CD40 agonist. The immature DCs may be contacted with PGE₂ at about the same time that they receive the first signal of an IFN-γR agonist and a TNF-αR agonist. In some methods, signaling is in the absence of an effective amount of IL-1β and/or IL-6. GM-CSF and at least one of IL-4 or IL-13 may be present in the medium at the time the dendritic cells receive the first and second signals.

Signaling with IFN-γ receptor agonists, TNF-α receptor agonists, and/or CD40 agonists can be accomplished by contacting a cell directly with IFN-γ polypeptides and/or proteins and/or TNF-α polypeptides or proteins and/or CD40 agonists, respectively. Similarly, IFN-γ and TNF-α receptor agonists can be aptamers, antibodies, and the like, that have a similar biological activity. Alternatively, signaling of a cell with IFN-γR agonists, TNF-αR agonists and/or CD40 agonists can occur upon translation of mRNA encoding such polypeptides or proteins within the dendritic cell. Such mRNA may be introduced into the cell by transfection or other means, and the signaling then occurs upon expression of the IFN-γR agonist, TNF-αR agonist and CD40 agonist polypeptides and/or proteins. Thus, signaling can be initiated by providing the signaling agonist in the culture medium, introduction of the agonist into the cell, and/or upon translation within the dendritic cell of an mRNA encoding an agonistic polypeptide. The methods can be practiced *in vivo* or *ex vivo.* Dendritic cells matured *ex vivo* according to the methods described herein can then be administered to the subject to induce or enhance an immune response along with the T_{SCM}s produced by coculturing with the DCs.

Dendritic cells can be further modified by the administration of an immunogen (*e.g.,* an antigen) to the DCs. The immunogen can be delivered *in vivo* or *ex vivo.* The immunogen can be delivered to the cells using methods known in the art, and can be delivered as polypeptides or proteins (*e.g.,* by "pulsing") or as nucleic acids encoding the immunogen (*e.g,* by transfection or electroporation). In some embodiments, the polynucleotide is an mRNA. In some methods of producing PME-CD40L DCs, the antigen-encoding mRNA is electroporated together with an mRNA encoding a CD40 agonist or substantially concurrent with CD40 agonist signaling.

As will be understood by one of skill in the art, PME-CD40L DCs can also be transfected with RNA encoding antigens from any pathogen or disease of interest; such antigens can be from one individual subject or multiple subjects and can be from a pathogen infection of the subject from which the antigens are isolated or from another subject. Consensus antigens and pathogen-specific antigens are known in the art and may also be used in these methods of preparing PME-CD40L DCs. The DCs will process the antigens and display the antigens on their cell surface; these mature DCs can be used to educate naive immune effector cells. RNA encoding antigens from a cancer and/or tumor sample removed from a subject may be used to transfect DCs in this manner. RNA encoding HIV antigens from a sample removed from a subject may also be used to transfect DCs.

For example, PME-CD40L DCs that were transfected with MART-encoding mRNA stimulated autologous CD8+ T cells to produce responder CD8+ T cells, as described, for example, in WO2006042177 (Healey et al.) and WO2007117682 (Tcherepanova et al.)*.* Also, PME-CD40L matured DCs loaded with total amplified Renal Cell Carcinoma ("RCC") tumor RNA induced a fully autologous CTL response (see WO2006042177 (Healey et al.)).

In some examples, the PME-CD40L DCs used in methods described herein to produce T_{SCM} cells are transfected with RNA encoding part or all of the HIV proteins Gag, Nef, Tat, and Rev, as described in WO2006031870 and U.S. Pub. No. 20080311155 (Nicolette et al.)*.* Briefly, DCs are transfected with RNA encoding one or more polypeptides from multiple strains of HIV present in an individual subject; the RNA is derived from nucleic acid amplification of pathogen polynucleotides. Primers to amplify such pathogen polynucleotides can be designed to compensate for sequence variability between multiple strains of said pathogen, for example, when said pathogen is HIV, as described in WO2006031870 and U.S. Pub. No. 20080311155. Such primers can include, for example, primers disclosed in WO2006031870, including forward and reverse primers for Gag, Nef, Tat, and Rev. The DCs resulting from this process have been shown to be capable of stimulating an immune response to HIV in HIV patients. In this manner, a DC vaccine autologous to a patient can be produced and used to stimulate an immune response to the HIV strains found in that patient.

PME-CD40L DCs can also be stored by contacting an enriched dendritic cell population with a suitable cryopreservative under suitable conditions and frozen (see, *e.g.,* WO 2002016560 and U.S. Pat. No. 8,574,901 (Schuler et al.)).

Many methods are known in the art for the isolation and expansion of various cells for *in vitro* expansion and differentiation into dendritic cells, including CD34⁺ stem cells (see for example, U.S. Pat. No. 5,199,942). The following descriptions are for the purpose of illustration only and in no way are intended to limit the scope of the invention.

CD34⁺ stem cells can be isolated from bone marrow cells or by panning the bone marrow cells or other sources with antibodies which bind unwanted cells, such as CD4⁺ and CD8⁺ (T cells) (see, *e.g.,* Inaba, et al. (1992) J. Exp. Med. 176: 1693-1702). Human CD34⁺ cells can be obtained from a variety of sources, including cord blood, bone marrow explants, and mobilized peripheral blood. Purification of CD34⁺ cells can be accomplished by antibody affinity procedures, for example, as described in Paczesny et al. (2004) J. Exp. Med. 199: 1503-11; Ho et al. (1995) Stem Cells 13 (suppl. 3): 100-105; Brenner (1993) Journal of Hematotherapy 2:7-17; and Yu, et al. (1995) PNAS 92: 699-703.

CD34⁺ stem cells can be differentiated into dendritic cells by incubating the cells with appropriate cytokines, as is known in the art. For example, human CD34⁺ hematopoietic stem cells can be differentiated *in vitro* by culturing the cells with human GM-CSF and TNF-α (see, *e.g.,* Szabolcs, et al. (1995) J. Immunol. 154: 5851-5861). Optionally, SCF or other proliferation ligand (*e.g.,* F1t3) is added. Dendritic cells can be isolated by florescence activated cell sorting (FACS) based on expression of cell surface markers or by other standard methods.

As is apparent to those of skill in the art, dose ranges for differentiating stem cells and monocytes into dendritic cells are approximate. Different suppliers and different lots of cytokine from the same supplier vary in the activity of the cytokine. One of skill can readily titrate each cytokine which is used to determine the optimal dose for any particular cytokine.

DCs can be generated from non-proliferating CD14⁺ precursors (monocytes) in peripheral blood by culture in medium containing GM-CSF and IL-4 or GM-CSF and IL-13 (see, *e.g.,* WO 97/29182; Sallusto and Lanzavecchia (1994) J. Exp. Med. 179: 1109 and Romani et al. (1994) J. Exp. Med. 180:83). In some instances, patients can be pretreated with cytokines such as G-CSF, but in most cases this is not necessary because CD14⁺ precursors are sufficiently abundant (Romani et al. (1996) J. Immunol. Methods 196: 137). Others showed that it is possible to avoid non-human proteins such as FCS (fetal calf serum), and to obtain fully and irreversibly mature and stable DCs by using autologous monocyte conditioned medium as maturation stimulus (see, *e.g.,* Romani et al. (1996) Immunol. Methods 196: 137; Bender et al. (1996) J. Immunol. Methods 196: 121). However, these studies did not result in mature DC having increased levels of IL-12 and/or decreased levels of IL-10 and thus did not produce PME-CD40L DCs.

In some examples, DCs used to produce T_{SCM} cells in methods described herein are derived from the same patient or subject; that is, the DCs and the T_{SCM} cells or their precursor cells are obtained from the same patient or subject (*i.e.,* they are autologous). In other examples, the DCs and the T_{SCM} cells are derived from different subjects (*i.e.,* they are allogeneic or heterologous).

In some methods of this disclosure, T cells are isolated from mammals and cocultured with PME-CD40L DCs *in vitro* to produce T_{SCM} cells. For example, in one such method, established procedures are used to separate PBMCs from red blood cells and neutrophils with Ficoll-Hypaque density gradient centrifugation. Cells are washed with modified AIM-V (which consists of AIM-V (GIBCO® Life Technologies) with 2 mM glutamine, 10 µg/ml gentamicin sulfate, 50 µg/ml streptomycin) supplemented with 1% fetal bovine serum (FBS). T cells are enriched by negative and/or positive selection with appropriate monoclonal antibodies coupled to columns or magnetic beads according to standard techniques and/or manufacturer or provider directions. An aliquot of cells is analyzed for cell surface phenotype including CD4, CD8, CD3 and CD14, and for the purpose of illustration only, cells are washed and resuspended at a concentration of about 5 x 10⁵ cells per ml of AIM-V modified as above and containing 5% FBS and 100 U/ml recombinant IL-2 (rIL-2) (referred to as "supplemented AIM-V"). The term "coculture" refers to a cell culture known to contain at least two different types of cells.

When cells are isolated from an HIV patient, HIV-infected cells may be preferentially removed from the population using reagents such as, for example, CD4-PE40 (*e.g.,* at 25nM). CD4-PE40 is a recombinant protein consisting of the HIV-1-binding CD4 domain linked to the translocation and ADP-ribosylation domains of *Pseudomonas aeruginosa* exotoxin A; it has been shown to inhibit p24 production in HIV-infected cell cultures and to selectively kill HIV-1-infected cells. To stimulate cell proliferation, OKT3 monoclonal antibody (Ortho Diagnostics™, Inc.) can be added.

T_{SCM} cells can be isolated from patient material; for example, T_{SCM} CTLs can be isolated from peripheral blood or tumor infiltrating lymphocytes. In some methods of the invention, T cells or PBMCs are isolated from human patients and cocultured with PME-CD40L DCs derived from the same patient or subject to produce and/or expand T_{SCM} cells *in vitro.* These T_{SCM} CTLs can then be used for adoptive transfer therapy by infusing them back into the same patient (autologous therapy) or into another patient (allogeneic therapy). Successful allogeneic adoptive transfer therapy of hematopoietic stem cells and lymphocytes has been reported, for example, in Cieri et al. ((2014) Immunol. Rev. 257: 165-180) and Kolb et al. ((1995) Blood 86: 2041-50).

T_{SCM} CTLs can be identified in co-cultures of T-cells and PME-CD40L DCs and isolated and/or expanded *in vitro.* If PME-CD40L DCs are loaded with antigen and used to expand a population of T_{SCM} cells, the resulting population will include T_{SCM} cells that are reactive to the antigen (for example, at least 30%, 40%, 50%, 60%, 70%, 80%, or 90% or more of the T_{SCM} cells produced by the expansion will be reactive to the antigen). This is demonstrated, for example, by the data provided in Figure 1 and discussed in Example 2, which illustrates the ability of PME-CD40L DCs loaded with the MART-1 tumor antigen to expand a population of MART-1-reactive T_{SCM} CTLs. In this manner, T_{SCM} CTLs can be produced that are reactive to any particular antigen or antigens; these can be used to enhance or stimulate the patient's immune response to the antigen by adoptive transfer therapy. For example, antigens can be prepared from a patient's own cancer cells and loaded into DCs that are used to expand a population of T_{SCM} CTLs that are then infused back into the patient. In some examples, antigens are prepared from an HIV patient (*i.e.,* a patient infected with HIV) and loaded into DCs that are used to expand a population of T_{SCM} CTLs that are infused back into the patient. Methods for preparing antigens from HIV patients and preparing DCs that present them are known in the art, for example, as described in WO2006031870 (Nicolette et al.) and discussed in more detail elsewhere herein.

T_{SCM} CTLs may also produce various cytokines, such as, for example, TNF-α or IFN-γ. The data shown in Figure 1 revealed that some of the MART-1+ T_{SCM} CTLs were "multi-functional," with 1.8% expressing TNF-α, 3.2% expressing CD107a, and 1.5% expressing IFN-γ; IL-2 expression was not detected. Methods are known in the art to separate cells based on particular functional attributes such as their expression of specific cytokines *(e.g.,* as discussed in Kammula et al. (1999) J. Immunol. 12: 6867-75 and Kammula et al. (2008) J. Transl. Med. 2008: 60), and cells can be selected on the basis of cytokine expression using a cytokine capture reagent *(e.g.,* as discussed in Brosterhus et al. (1999) Eur. J. Immunol. 12: 4053-59).

In another aspect, cell surface markers can be used to identify and/or isolate cells for various purposes. For example, DCs can be distinguished from other cells because they express MHC molecules and costimulatory molecules (*e.g.,* B7-1 and B7-2) and lack markers specific for granulocytes, NK cells, B cells, and T cells. T_{SCM} CTLs have some markers in common with naive cells such as, for example, CD27, CD28, and CD45RA. However, since T_{SCM} cells have been exposed to antigen they also express activation markers such as, for example, CD95 and CD122. T_{SCM} cells can be CD4+ or CD8+, and can also be CCR7+. The expression of markers facilitates identification, purification, and separation of these cells from other cells expressing at least one different marker; any suitable combination of markers may be used and is readily determined by one of skill in the art. Negative marker or cell selection may also be used. In this manner, the disclosure provides methods of identifying and/or separating, isolating, or enriching T_{SCM} cells from other cells on the basis of expression of one or more of CD27, CD28, CD45RA, CD95, CD122, CD4, CD8, CCR7, and PD-1. In some embodiments, T_{SCM} cells enriched as cells that are positive for CD27, CD28, and CD45RA. In some embodiments, T_{SCM} cells are further enriched as positive for CD8, CD95, CD28, CD7, and CD45RA. T_{SCM} cells may also be identified and/or separated or enriched from other cells based on their expression of PD-1.

Cells can be isolated and/or characterized by flow cytometry methods such as FACS analysis as well as by any suitable method known in the art, including but not limited to methods such as column chromatography, Western blots, radiography, electrophoresis, capillary electrophoresis, high performance liquid chromatography (HPLC), thin layer chromatography (TLC), hyperdiffusion chromatography, and the like, and various immunological methods such as fluid or gel precipitin reactions, immunodiffusion (single or double), immunoelectrophoresis, radioimmunoassays (RIAs), enzyme-linked immunosorbent assays (ELISAs), immunofluorescent assays, and the like.

Labeling agents which can be used to label cell antigens (including cell surface markers) include but are not limited to monoclonal antibodies, polyclonal antibodies, proteins, or other polymers such as affinity matrices, carbohydrates or lipids. Detection proceeds by any known method, such as immunoblotting, Western blot analysis, tracking of radioactive or bioluminescent markers, capillary electrophoresis, or other methods which track a molecule based on size, charge or affinity.

T_{SCM} CTLs can be identified by multi-color flow cytometry as cells that are positive for (*i.e.,* express at detectable levels) the markers CD8, CD95, CD28, CCR7, and CD45RA (also designated "CD8+/CD95+/CD28+/CCR7+/CD45RA+"). For subsequent use *in vivo* or *in vitro,* T_{SCM} CTLs can also be enriched, isolated or purified from other cells using magnetic bead isolation of cells having one or more of these markers, or in some instances it may be preferable to remove other cell types from a population including T_{SCM} cells using appropriate markers.

Cell separation methods based on the expression of surface markers are known in the art and include the use of magnetic bead isolation, FACS sorting (*e.g.,* as discussed in Basu et al. (2010) J. Vis. Exp. 41), and microelectromechanical systems chips ("MEMS" chips)-based sorting *(e.g.,* as discussed in Shoji and Kawai (2011) Top. Curr. Chem. 2011: 1-25). FACS machines and cell sorters are commercially available (*e.g.,* the BD Bioscience LSRII and the BD FACSAria) and can be used according to manufacturer's instructions.

Cells can be isolated or separated from other cells by positive or by negative selection where appropriate, or by both positive and negative selection. For example, T_{SCM} cells can be enriched from a population including other cells such as PBMCs or lymphocytes using negative selection to deplete other cell types followed optionally by positive selection for CD8 and/or CD4 and positive selection for CD95. Kits and reagents are known in the art for a variety of purification steps, allowing one of skill in the art to isolate or purify a known cell type; for example, Invitrogen's Dynabeads® Untouched™ Human T cell kit is designed to deplete human B cells, NK cells, monocytes, macrophages, platelets, dendritic cells, granulocytes, and erythrocytes using antibodies including mouse IgG antibodies against non-T cells: human CD14, CD16, CD19, CD36, CD56, CDw123, and CD235a. It will be appreciated from this example that one of skill in the art is capable of selecting particular (often commercially-available) antibodies and selection tools to enrich and/or deplete known cell types from a population of cells.

Selection for cells bearing particular markers can be performed for one marker at a time or for more than one marker at a time (*e.g.,* as discussed in Stemberger et al. (2012) PLoS One 4:e35798). Selection can also be performed serially, and different types of selection can be used on a particular group or population of cells in subsequent selection steps to obtain a desired subpopulation. Cells can also be selected based on their antigen specificity directly by isolating T cells reactive to HLA-peptide complexes (e.g., as discussed in Keenan et al. (2001) Br. J. Haematol. 2: 428-34). Cell markers that are useful for identification, screening, and/or selection include CD4, CD8, CD27, CD28, CD38, CD57, PD-1, HLA-DR, CD45RA, and CD95.

T_{SCM} CTLs can be expanded *in vitro* with PME-CD40L DCs presenting the antigen(s) of interest to produce a population of T_{SCM} CTLs reactive to a particular antigen or set of antigens. The expansion of the cells can be performed before or after isolation of the T_{SCM} cells from other cells, or both before and after (so that the T_{SCM} cells are purified or enriched both before the expansion step and after the expansion step). Methods of expanding T cells are known in the art, such as methods making use of IL-15 (see, *e.g.,* Klebanoff et al. (2004) Proc. Nat'l. Acad. Sci. USA 7: 1969-74) and IL-21 (see, *e.g.,* Albrecht et al. (2011) Cancer Immunol. Immunother. 2: 235-48). In some methods, cell developmental pathway modulators such as, for example, rapamycin can also be used to promote memory CD8+ T cell formation (see, *e.g.,* Rao et al. (2010) Immunity 1: 67-78).

T cells with particular antigen receptors can also be generated using methods of genetic modification, for example, using lentiviral vectors as described in Wang et al. ((2012) J. Immunother. 35: 689-701) and Terakura et al. ((2012) Blood 1: 72-82), or chimeric antigen receptors as discussed in Barrett et al. ((2014) Ann. Rev. Med. 65: 333-47). Unfortunately, lentiviral transduced cells are not suitable for therapeutic purposes, and proviral integration into the genome of the transduced cell can result in leukemia; thus, alternative methods are preferred.

In a clinical trial, HIV patients were treated with multiple doses of AGS-004 and the anti-HIV immune response was determined post therapy (see schematic diagram in Figure 3). "AGS-004" refers to PME-CD40L DCs containing "GNVR", the RNA antigen payload encoding the antigens Gag (G), Nef (N), Vpr (V), and Rev (R) (also called "GNVR DCs") (as described, for example, in WO2006031870). AGS-004 was found to increase the number of T_{SCM} cells in treated patients and to stimulate the immune response (see, e.g., Example 3 and Example 5, and data shown in the figures).

T_{SCM} CTLs can be detected or identified, for example, in co-cultures containing PBMCs and PME-CD40L DCs *in vitro* as well as in patient blood or other tissues. For example, multi-color flow cytometry detected T_{SCM} cells among PBMCs of HIV patients treated with PME-CD40L DCs encoding HIV antigens (GNVR DCs). Proliferating T_{SCM} CD4+ and CD8+ T cells were detected in PMBCs collected from HIV subjects receiving AGS-004 after *in vitro* stimulation with PME-CD40L DCs encoding HIV antigens. Viral load data and immune monitoring data from HIV patients was analyzed to determine the relationship between time to viral rebound after anti-retroviral therapy (ART) interruption and the anti-GNVR T cell response. Subjects on ART have no detectable HIV viral load, but once drug therapy is withdrawn the virus will typically rebound. A delay in viral rebound is indicative of an anti-HIV immune response.

CFSE can be used in conjunction with other cell markers to identify cell types that are proliferating. The frequency of CFSElo T cells represents the percentage of T cells proliferating *in vitro* after restimulation with GNVR DCs. Single time point data for the frequency of CFSElo T cells was analyzed at visit 9 and visit 13 post AGS-004 treatment, but neither time point showed an association between the percentage of proliferating T cells and extended time to viral rebound in the patient or reduction in viral load. However, surprisingly the inventors discovered that there was a positive association between favorable response to AGS-004 and increases in T_{SCM} cells for both the CD4+ and CD8+ T cell populations (identified as proliferating T cells expressing CD27, CD28, and CD45 RA; also designated CD27⁺/CD28⁺/CD45RA⁺). Four patients with proliferating CD27⁺/CD28⁺/CD45RA⁺ CD4 T_{SCM} cells (Figure 2A) and CD27⁺/CD28⁺/CD45RA⁺ CD8 T_{SCM} cells (Figure 2C) displayed a longer time to viral rebound after ART interruption (ATI) and/or a reduced viral load than the other group of patients. That is, the four patients with a favorable response (*e.g.,* an extended time to viral rebound and/or reduction in viral load) compared to the other three subjects had a marked difference in the percent change of proliferating CD27⁺/CD28⁺/CD45RA⁺ CD4 T_{SCM} cells (Figure 2B) and CD27⁺/CD28⁺/CD45RA⁺ CD8 T_{SCM} cells (Figure 2D) measured from baseline (visit 3) and after administration of AGS-004 determined as the mean response measured by averaging the response detected at visit 9 and visit 13.

Thus, T_{SCM} cells induced *in vivo* after treatment with PME-CD40L DC are present *in vitro* for downstream isolation and expansion. Further, the correlation between T_{SCM} cells and favorable response to AGS-004 makes the frequency and/or change in T_{SCM} cells in a patient a useful indicator of immune response in a patient. In this manner, the frequency and/or change in T_{SCM} cells in a patient following a treatment (for example, with PME-CD40L DCs as in the AGS-004 clinical trial) is a valuable tool for assessing a patient's likely clinical outcome. In some examples, T_{SCM} cells disclosed herein have low or no expression of PD-1, a marker associated with dysfunctional CD4 and CD8 T cell activation. In this manner, expression of PD-1 can serve as an indicator of immune response.

By monitoring the frequency and/or change in T_{SCM} cells in a patient (and/or the expression of PD-1 by those cells), it is possible to predict or determine whether a treatment of a patient has been or will be effective in inducing an immune response as measured, *e.g.,* by a decrease in viral load or an extended time to viral rebound. Similarly, by monitoring the frequency and/or change in T_{SCM} cells in a patient, it is also possible to evaluate when a treatment has been effective and/or when a patient has had enough doses of a treatment (such as, for example, AGS-004) to be effective in inducing an immune response. In some instances, an increase of at least 20%, 30%, 40%, 50%, 60%, 100%, or 200% or more of T_{SCM} cells in a patient will indicate that the patient has had a sufficient immune response that a treatment (*e.g.,* treatment with AGS-004) has reached a treatment threshold and may properly be discontinued. In some instances, such an increase in T_{SCM} cells in a HIV patient will indicate that STI may continue; that is, that ART is not required for effective treatment. Conversely, if such an increase in T_{SCM} cells is not present, effective treatment may require that the STI end and ART be resumed. Such treatment decisions are within the skill of a clinician with the guidance of known measures of patient health and also by changes in patient T_{SCM} cell level as described herein. In this manner, the present disclosure provides methods of determining whether a treatment has been effective and/or whether a particular treatment should be continued or discontinued.

In some examples, methods of determining or confirming effective treatment of a patient for an immune-related disease or disorder comprise obtaining an aliquot of blood from the patient; quantifying the number of T_{SCM} cells present in the patient's blood; administering a treatment to said patient comprising autologous mature DCs prepared *in vitro;* quantifying the number of T_{SCM} cells present in the patient's blood; and confirming that the number or frequency of T_{SCM} cells present in the patient's blood has reached a treatment threshold, such as, for example, an increase in at least 50%, 100%, 150%, or 200% of T_{SCM} cells present in the patient's blood, where the treatment threshold indicates that a treatment has been effective and may be discontinued. Conversely, if the number or frequency of T_{SCM} cells in the patient's blood has not reached the treatment threshhold, additional treatment(s) are indicated, such as, for example, additional doses of AGS-004 and/or the need to resume Anti-Retroviral Therapy ("ART"). Other treatment thresholds or measures can also be used, such as, for example, HIV RNA assays. In some examples, the treatment threshold is an increase in T_{SCM} cells that are proliferating and/or in Tscm cells that have low or no expression of PD-1. Similarly, in some examples an increase in T cells and/or T_{SCM} cells expressing PD-1 indicates that an immune response is defective or ineffective. For example, in a clinical trial of AGS-004 in HIV-infected patients, during the STI phase of treatment, an increase in the population of CD8+ CD38+ CD57⁻ PD-1+ HLA-DR⁻ cells after 8 weeks of STI was found to be inversely correlated with the duration of STI. In this manner, T_{SCM} cells can serve as an indicator or measure of a patient's immune response. In some examples, the desired result of a treatment is that the immune response has been stimulated so that an increase in T_{SCM} cells can be measured (that is, it is above the level of detection, such as at least 10%, 20%, or 30% or more); in these examples, a treatment is determined to be effective if it results in such an increase.

Also disclosed herein are methods of measuring an immune response in a patient having a disease or disorder, comprising the steps of: obtaining a sample of the patient's blood for determining the quantity and/or frequency of T_{SCM} cells present therein; administering autologous mature DCs prepared *in vitro* to said patient; subsequently obtaining a sample of the patient's blood for determining the quantity and/or frequency of T_{SCM} cells present therein post-treatment; and comparing the quantity and/or frequency of T_{SCM} cells present in the patient's blood post-treatment to the quantity prior to treatment, wherein an increase of Tscm cells indicates that an immune response has been induced in the patient.

Also disclosed herein are are methods of making a recommendation for treatment of a patient, comprising the steps of: quantifying the number of T_{SCM} cells present in a sample of a patient's blood to establish a baseline reading; following administration to said patient of a treatment comprising autologous mature DCs prepared *in vitro,* quantifying the number of T_{SCM} cells present in a sample of said patient's blood to establish a post-treatment reading; comparing said baseline reading and said post-treatment reading to determine whether the frequency or amount of T_{SCM} cells present in the sample of the patient's blood has increased to meet the treatment threshhold; and making a recommendation to continue treatment of said patient if said treatment threshhold was not met or to discontinue or suspend treatment of said patient if the treatment threshhold was met.

Also disclosed herein are methods of evaluating whether an immune response was induced in a patient by a treatment, comprising the steps of: obtaining a first sample of a patient's blood; labelling the cells from said sample to detect the presence of cell surface markers CD27, CD28, and CD45RA; counting the number of cells in the sample that are positive for CD27, CD28, and CD45 RA; following administration to said patient of a treatment, obtaining a second sample of said patient's blood; labelling the cells from said second sample to detect the presence of cell surface markers CD27, CD28, and CD45RA; counting the number of cells in said second sample that are positive for CD27, CD28, and CD45 RA; and determining whether there has been an increase or decrease in the number or proportion of cells that are positive for CD27, CD28, and CD45RA in said first and second sample; wherein an increase indicates that an immune response was induced in the patient. In some examples of these methods, the treatment is the administration to the patient of autologous mature DCs that were produced *in vitro.*

Aslo disclosed herein is a method of stimulating immune effector cells, comprising culturing said cells in the presence of PME-CD40L DCs to produce stimulated immune effector cells. In another example, the disclosure provides a method of enhancing immunity in a subject comprising administering to the subject an effective amount of such stimulated immune effector cells (T_{SCM} cells). Introducing or administering immune cells into a subject is generally referred to as adoptive transfer therapy and is intended to help stimulate the subject's immune response. Adoptive transfer therapy is known in the art and has been demonstrated in a number of studies, such as, for example, Cobbold et al. (2005) J. Exp. Med. 3: 379-86 and Schmitt et al. (2011) Transfusion 3: 591-99.

The compositions described herein are useful to raise an immune response in a subject by administering to the subject an effective amount of the enriched population of cells, e.g., T_{SCM} cells. The cells can be allogeneic (heterologous) or autologous to the subject. They can be administered to a subject to raise or induce an immune response in a subject in a method comprising administering to the subject an effective amount of the enriched populations as described above. The educated effector cells can also be used to enhance immunity in a subject by delivering to the subject an effective amount of these cells. For example, T_{SCM} cells can be administered to a patient infected with HIV to increase the immune response and/or to help inhibit opportunistic infection(s), or T_{SCM} cells can be administered to a cancer patient to increase the immune response to the cancerous cells. In such examples, an effective amount of T_{SCM} cells is one that increases any measure of immune response by a statistically significant amount.

The disclosure also provides *in vitro* methods involving T_{SCM} cells. For example, dendritic cells loaded with an antigen can be used to produce and expand T_{SCM} cells *in vitro.* T_{SCM} cells educated *in vitro* (*e.g.,* by coculturing with DCs presenting antigen) can be introduced into a mammal where they are cytotoxic against target cells bearing antigenic peptides corresponding to those the T cells are activated to recognize. These target cells are typically cancer cells, or infected cells which express unique antigenic peptides on their MHC class I surfaces; thus, for example, target cells can be cells infected with HIV or can be cancer cells.

T_{SCM} cells produced by the methods of this invention can be administered directly to the subject (*e.g.,* a human patient) to produce T cells active against a selected immunogen. The cells are administered in any suitable manner, for example, with pharmaceutically acceptable carriers, which are well known in the art. Thus, for example, the cells can be provided in a composition also comprising a pharmaceutically acceptable carrier. In this manner, disclosed herein is a medicament comprising T_{SCM} cells for use in treating a patient. Similarly, the T_{SCM} cells disclosed herein are suitable for use in preparing a medicament for treating a patient, such as a cancer patient or a patient infected with HIV. Suitable methods of administering cells in the context of the present disclosure to a subject are available, and, although more than one route can be used to administer a particular cell compositon, a particular route can often provide a more immediate and effective reaction than another route. Administration can be by methods known in the art to successfully deliver a cell into ultimate contact with a subject's blood or tissue cells. Preferred routes of administration include but are not limited to intradermal and intravenous administration.

Pharmaceutically acceptable carriers are determined in part by the particular composition being administered, as well as by the particular method used to administer the composition. Accordingly, there is a wide variety of suitable formulations of pharmaceutical compositions of the present disclosure. Most typically, quality controls (microbiology, clonogenic assays, viability tests), are performed and the cells are infused into the patient from whom they were derived and/or isolated, preceded by the administration of diphenhydramine and hydrocortisone. See, for example, Korbling et al. (1986) Blood 67:529-532 and Haas et al. (1990) Exp. Hematol. 18:94-98. The dose of cells (*e.g.,* T_{SCM} cells) administered to a subject is in an amount effective to achieve the desired beneficial therapeutic response in the subject over time, or to inhibit growth of cancer cells, or to inhibit infection (*i.e.,* an "effective amount"); those of skill in the art recognize however that the patient can benefit from an increase in any measure of the immune response, even if a complete cure is not achieved.

For the purpose of illustration only, a method of adoptive transfer therapy of the disclosure can be practiced by obtaining and saving blood samples from the patient prior to infusion for subsequent analysis and comparison. Generally, at least about 10⁴ to 10⁶ and typically between 1 x 10⁸ and 1 x 10¹⁰ cells can be infused (*e.g.,* intravenously or intraperitoneally) into a 70 kg patient over roughly 60-120 minutes. Vital signs and oxygen saturation by pulse oximetry can be closely monitored, and blood samples obtained at intervals following infusion (*e.g.,* 5 minutes and 1 hour) and saved for analysis. Cell re-infusions can be repeated roughly every month for a total of 10-12 treatments in a one year period, if deemed appropriate. After the first treatment, infusions can be performed on an outpatient basis at the discretion of the clinician. If the re-infusion is given as an outpatient treatment, the participant is monitored for at least 4 hours following the treatment.

For administration, cells of the present disclosure can be administered at a rate determined by the effective dose, the LD-50 of the cell type (or other measure of toxicity), and the side-effects of the cell type at various concentrations, as applied to the mass and overall health of the subject. Administration can be accomplished via single or divided doses. The cells of this disclosure can supplement other treatments for a condition by known conventional therapy, including cytotoxic agents, nucleotide analogues and biologic response modifiers. Similarly, biological response modifiers are optionally added for treatment; for example, the cells are optionally administered with an adjuvant, or cytokine such as GM-CSF, IL-12 or IL-2.

The IFN-γR agonist used in the PME-CD40L process can be IFN-γ or a biologically active fragment thereof. Preferably, the IFN-γ is a mammalian IFN-γ, most preferably a human IFN-γ. The cDNA and amino acid sequence of human IFN-γ are shown in SEQ ID NOs: 5 and 6 of WO2007117682, respectively. Preferably, the IFN-γ has the sequence shown in SEQ ID NO:6, or a fragment thereof. In one example, the IFN-yR comprises a polypeptide having at least 80% sequence identity with SEQ ID NO:6 of WO2007117682. Preferably, the IFN-γR agonist has at least 85%, 90%, 95%, 97%, 98% or 99% sequence identity with SEQ ID NO:6 of WO2007117682. Methods for testing the activity of IFN-γR agonists are known in the art (see, for example, Magro et al. (2004) Br. J. PharmacoL 142: 1281-92). Immature DCs can be signaled by adding an IFN-γR agonist the culture medium, or by expressing the IFN-γR agonist in the dendritic cell. In some examples, the DC is transfected with an mRNA encoding an IFN-γR agonist, such as SEQ ID NO:6 of WO2007117682, or a biologically active fragment thereof. Signaling would then occur upon translation of the mRNA within the dendritic cell. Most preferably, the IFN-γR agonist is added to the culture medium containing immature DCs. In a preferred example, the culture medium further comprises PGE₂ and/or GM-CSF plus IL-4 or IL-13.

The second signal used to produce PME-CD40L DCs is a transient signal with a CD40 agonist. The signal can be considered transient if the DCs are loaded with an mRNA encoding a CD40 agonist, or if medium containing a CD40 agonist is removed from the DCs. Thus, persistent expression of a CD40 agonist polypeptide, such as constitutive expression of CD40L from a lentiviral vector, is not considered transient expression. The CD40 agonist signal can also be considered transient if the DCs are loaded/transfected with RNA or with an expression vector encoding a CD40 agonist, provided that either: 1) the promoter driving CD40 agonist expression is not constitutive in DCs, or 2) the expression vector does not integrate into the DC genome or otherwise replicate in DCs.

In some methods, the CD40 agonist is a CD40L polypeptide or a CD40 agonistic antibody. In general, ligands that bind CD40 may act as a CD40 agonist, for example, a CD40 agonist can be an aptamer that binds CD40. Preferably, the CD40 agonist is delivered as mRNA encoding CD40L. Administration of the second signal comprising CD40L to the cells by transfection of immature or mature DCs with CD40L mRNA produces the modified PME-CD40L DCs that induce immunostimulatory responses rather than immunosuppressive ones.

In some methods used to produce PME-CD40L DCs, CD40L-mRNA-transfected dendritic cells are cultured in medium containing IFN-γ (and optionally PGE₂) immediately after transfection and thus prior to translation of the CD40L mRNA to produce an effective amount of a CD40L signal. In this situation, although IFN-γ is added after transfection with CD40L mRNA, the dendritic cells receive the IFN-γ signal prior to the signal that results from the translation of the CD40L mRNA. Thus, the order in which the agents are delivered to the cells is important only in that CD40L signaling must occur after IFN-γ signaling. In these methods, the signaling of the DCs can occur *in vivo* or *ex vivo,* or alternatively one or more signaling step may occur *ex vivo* and the remaining steps of the method can occur *in vivo.*

As used herein, "CD40 Ligand" (CD40L) encompasses any polypeptide or protein that specifically recognizes and activates the CD40 receptor and activates its biological activity. The term includes transmembrane and soluble forms of CD40L. In preferred examples, the CD40 agonist is a mammalian CD40L, preferably a human CD40L. A human CD40L cDNA and the corresponding amino acid sequence are shown in SEQ ID NOs:1 and 2 of WO2007117682, respectively.

In some methods used to prepare PME-CD40L DCs, the method comprises the sequential steps of: (a) signaling isolated immature dendritic cells (iDCs) with a first signal comprising an interferon gamma receptor (IFN-γR) agonist and a TNF-αR agonist, to produce IFN-yR-agonist-signaled dendritic cells; and (b) signaling said IFN-γR-agonist-signaled dendritic cells with a second transient signal comprising an effective amount of a CD40L polypeptide to produce CD83⁺ CCR7⁺ mature dendritic cells, wherein the CD40L polypeptide consists essentially of amino acid residues 21-261 of SEQ ID NO:2 of WO2007117682 or a polypeptide having at least 80% sequence identity to amino acid residues 21-261 of SEQ ID NO:2 of WO2007117682.

In some methods used to prepare PME-CD40L DCs, the method comprises the sequential steps of: (a) culturing isolated immature dendritic cells (iDCs) with an interferon gamma receptor (IFN-γR) agonist in the presence of a TNF-αR agonist and PGE₂ for approximately 12 to 30 hours to produce CD83⁺ mature dendritic cells; and (b) approximately 12 to 30 hours after initiating step (a), transfecting said CD83⁺ mature dendritic cells (mDCs) with mRNA encoding a CD40L polypeptide consisting of amino acid residues 21-261 of SEQ ID NO:2 of WO2007117682 and an mRNA encoding one or more antigens to produce CD83⁺ CCR7⁺ mature dendritic cells.

CD40 Ligand was cloned in 1993 and reported by Gauchat et al. (1993) FEBS Lett. 315: 259. Shorter soluble forms of the cell-associated full-length 39 kDa form of CD40 Ligand have been described with molecular weights of 33 kDa and 18 kDa (Graf et al. (1995) Eur. J. Immunol. 25: 1749; Ludewig et al. (1996) Eur. J. Immunol. 26: 3137; Wykes et al. (1998) Eur. J. Immunol. 28: 548). The 18 kDa soluble form generated via intracellular proteolytic cleavage lacks the cytoplasmic tail, the transmembrane region, and parts of the extracellular domain, but conserves the CD40 binding domain and retains the ability to bind to CD40 receptor; therefore, it is an example of a CD40-receptor-signaling agent. See Graf *et al.* (1995) supra. U.S. Patent Nos. 5,981,724 and 5,962,406 also disclose DNA sequences encoding human CD40 Ligand (CD40L), including soluble forms of CD40L.

The open reading frame for CD40L is represented by nucleotides 40 to 822 of SEQ ID NO.1 of WO2007117682, while the TGA stop codon is at position 823 to 825. In any of the CD40L polynucleotide sequences used to produce PME-CD40L DCs, a sequence containing a silent mutation may be used; for example, a variant due to codon degeneracy of the 102^{nd} codon in the CD40L sequence (nucleotides 343 to 345 of SEQ ID NO:1 of WO2007117682) changes the "AAA" codon to an "AAG" codon, both of which code for Lys. Also useful in methods to produce mature DCs are truncated CD40L (residues 47 to 261 of SEQ ID NO:2 of WO2007117682, encoded by nucleotide residues 178 to 825 of SEQ ID NO:1 of WO2007117682) and CD40L fragments encoded by nucleotides 43 to 825 of said SEQ ID NO:1, 181 to 825 of said SEQ ID NO:1, 193 to 825 of said SEQ ID NO:1, 376 to 825 of said SEQ ID NO:1, 379 to 825 of said SEQ ID NO:1 and 400 to 825 of said SEQ ID NO:1 of WO2007117682. In some methods of maturation, the CD40L polypeptide is a polypeptide comprising the sequence set forth in SEQ ID NO:2 of WO2007117682. However, any polypeptide fragment of the full-length CD40L (or DNA or RNA encoding it) may be used in the methods if the polypeptide acts as a CD40 ligand by specifically binding CD40 and producing biological activity.

In some methods of DC maturation, the CD40L polypeptide is encoded by an mRNA comprising a polynucleotide encoding CD40L and further comprising a 3' untranslated sequence known in the art such as the CD40 receptor 3' UTR, the untranslated region of the final exon of the human beta-actin 3' UTR, the minimal functional element of the human beta-actin 3' UTR, and the simian rotavirus gene 6 3' UTR. The mRNA may also, or alternatively, comprise a 5' untranslated sequence known in the art such as the human Hsp70 5' UTR, the mouse VEGF 5' UTR, the minimal functional element of the mouse VEGF 5' UTR, the spleen necrosis virus LTR RU5 region, and the tobacco etch virus 5' leader sequence. Preferably, these RNAs are capped and polyadenylated.

The CD40 receptor can also be activated by use of CD40 agonist antibodies, antibody fragments, derivatives and variants thereof, which are known in the art. CD40 agonist antibodies can be purchased from commercial vendors such as Mabtech (Nacka, Sweden). The literature also provides examples otf CD40 agonist antibodies and antibody fragments. See, *e.g.,* Osada *et al.* (2002) 25(2): 176 and Ledbetter et al. (1997) Crit. Reviews in Immunol. 17: 427. Modified CD40L can also be used in methods of dendritic cell maturation; for example, CD40L includes polypeptides that have been altered through addition, subtraction, or substitution, either conservatively or non-conservatively, of any number of amino acids, provided that the resulting protein binds CD40 on the surface of DCs.

Steps of the methods described herein can be practiced *in vivo* or *ex vivo,* as appropriate. When practiced *ex vivo,* the method can be practiced in an open or closed system. Methods and systems for culturing and enriching cell populations are known in the art. See Examples 1 and 2 of U.S. Patent Publication No. 2004/0072347; see also U.S. Patent Publication No. 2003/0235908, which describes closed systems for cell expansion.

The methods of preparing mature DCs can be further modified by contacting the cell with an effective amount of a cytokine or co-stimulatory molecule, *e.g.,* GM-CSF, IL-4 and PGE₂. In exampleswhere the immature DCs are signaled with a TNFaR agonist followed by signaling with CD40 agonist, effective amounts of IL-1β and/or IL-6 are specifically excluded from the culture.

The method used to produce PME-CD40L DCs can also include delivering to the immature or mature DCs an effective amount of an antigen which will be then be processed and presented by the mature DCs. Antigens can be naturally occurring or recombinantly produced. The antigens can be delivered to the cells as polypeptides or proteins or as nucleic acids encoding them using methods known in the art. In some methods, one or more polynucleotides encoding one or more antigens are introduced into the iDCs, signaled DCs or CCR7⁺ mature DCs by methods known to those of skill in the art such as electroporation. Most preferably, the polynucleotide is an mRNA. In preferred examples, the antigen or antigen-encoding mRNA is introduced together with an mRNA encoding a CD40 agonist or substantially concurrent with CD40 agonist signaling.

In methods where the antigen is delivered as a polynucleotide or gene encoding the antigen, expression of the gene results in antigen production either in the individual being treated (when delivered *in vivo*) or the cell culture system (when delivered *in vitro*); techniques for doing so are known in the art. Preferably, an mRNA encoding the antigen is introduced into the DC, and may be cotransfected with an mRNA encoding a CD40L polypeptide.

Methods of loading dendritic cells with antigens are known to those of skill in the art. In one example, the dendritic cells are cultured in medium containing the antigen. The DCs then take up and process the antigen on the cell surface in association with MHC molecules. Preferably, the DCs are loaded with antigen by transfection with a nucleic acid encoding the antigen, for example, an mRNA. Methods of transfecting DCs are known to those of skill in the art.

An antigen can be a single known antigen or can be a collection of antigens. A collection of antigens may come from one particular source, such as for example a patient's cancer cells or HIV-infected cells, or may come from several sources, such as for example HIV-infected cells from several different patients. Antigens for use in methods of producing PME-CD40L DCs include, but are not limited to, antigens from: pathogens, pathogen lysates, pathogen extracts, pathogen polypeptides, viral particles, bacteria, proteins, polypeptides, cancer cells, cancer cell lysates, cancer cell extracts, and cancer-cell-specific polypeptides. For example, antigens that can be used to produce PME-CD40L DCs include such well-known antigens as MART-1; the resulting PME-CD40L DCs presenting MART-1 can then be used to expand populations of MART-1-reactive T_{SCM} CTLs (see, *e.g.,* data shown in Figure 1 and discussed in Example 2).

Thus, the methods described herein further comprise introducing into iDCs, signaled DCs or CCR7⁺ mature DCs one or more antigens or a polynucleotide(s) encoding one or more antigens to produce an antigen-loaded CCR7⁺ mature DCs. The antigen or antigen-encoding polynucleotide (*e.g.,* mRNA) can be introduced: prior to said first signal; subsequent to said first signal and prior to said second signal; or subsequent to said second signal or substantially concurrent with said second signal, or during more than one of these intervals. mRNA encoding one or more antigens can be transfected into cells at the same time as other mRNA, such as mRNA encoding CD40L, or at a different time.

The antigen may be delivered in its "natural" form in that no human intervention was involved in preparing the antigen or inducing it to enter the environment in which it encounters the DC. Alternatively or additionally, the antigen may comprise a crude preparation, for example of the type that is commonly administered in a conventional allergy shot or the antigen may comprise a tumor lysate. The antigen may alternatively be substantially purified, *e.g.,* at least about 90% purified or isolated.

An antigen that is a peptide may be generated, for example, by proteolytic cleavage of isolated proteins using methods known in the art, or may be chemically synthesized, *e.g.,* on a commercially-available, automated synthesizer. Also, recombinant techniques may be employed to create a nucleic acid encoding the peptide of interest, and to express that peptide under desired conditions.

The antigen can alternatively have a structure that is distinct from any naturally-occurring compound. In certain examples of the disclosure, the antigen is a "modified antigen" having a structure that is substantially identical to that of a naturally-occurring antigen but that includes one or more deviations from the exact structure of the naturally-occurring compound. For instance, where the naturally-occurring antigen is a protein or polypeptide antigen, a modified antigen as compared with that protein or polypeptide antigen may have an amino acid sequence that differs from that of the naturally-occurring antigen in the addition, substitution, or deletion of one or more amino acids, and/or might include one or more amino acids that differ from the corresponding amino acid in the naturally-occurring antigen by the addition, substitution, or deletion of one or more chemical moieties covalently linked to the amino acid. In some instances, the naturally-occurring and modified antigens share at least one region of at least 5 amino acids that are at least approximately 75% identical. The antigens can also be modified by linking a portion of sequence from a first polypeptide (*e.g.,* a first antigen) to a portion of sequence from a second polypeptide (*e.g.,* a second antigen, a signal sequence, a transmembrane domain, a purification moiety, *etc*.) by means of a peptide bond. Those of ordinary skill in the art will appreciate the diversity of such fusion proteins for use in accordance with the present disclosure.

The amount of antigen to be employed in any particular composition or application will depend on the nature of the particular antigen and of the application for which it is being used, as will readily be appreciated by those of skill in the art, and can be adjusted by one of skill in the art to provide the necessary amount of expression.

Where the antigen is a fragment, it may be generated, for example, by proteolytic cleavage of isolated proteins. Any of a variety of cleavage agents may be utilized including, but not limited to, pepsin, cyanogen bromide, trypsin, chymotrypsin, *etc.* Alternatively, peptides may be chemically synthesized, preferably on an automated synthesizer such as is available in the art (see, for example, Stewart et al., (1984) Solid Phase Peptide Synthesis, 2d. Ed., Pierce Chemical Co.). Also, recombinant techniques may be employed to create a nucleic acid encoding the peptide of interest, and to express that peptide under desired conditions (*e.g.,* in a host cell or an *in vitro* expression system from which it can readily be purified).

In preferred embodiments, the antigen is from a cancer cell or a pathogen. The cancer cell can be any type of cancer cell, including a renal cancer cell (*e.g.,* from renal cell carcinoma), a multiple myeloma cell or a melanoma cell. Preferred pathogens include HIV and HCV. In preferred embodiments, the antigen is delivered to the DCs in the form of RNA isolated or derived from a cancer cell or a pathogen or pathogen-infected cell (*e.g.,* an HIV-infected cell). Methods for RT-PCR of RNA extracted from any cell (*e.g.,* a cancer cell or pathogen cell), and *in vitro* transcription are disclosed in WO2006031870 (Nicolette et al.) and U.S. Pub. 20070248578 (Tcherepanova et al.)*.*

Where both cytokine and antigen are to be delivered to an individual, they may be provided together or separately. When they are delivered as polypeptides or proteins, they can be delivered in a common encapsulation device or by means of physical association (*e.g.,* by covalent linkage). Similarly, the compounds can be provided together when polynucleotides encoding both are provided; for example, genes for both may be provided as part of the same nucleic acid molecule. In some examples, this nucleic acid molecule may be prepared so that both factors are expressed from a single contiguous polynucleotide, for example as a fusion protein in which the cytokine and the antigen are covalently linked to one another via a peptide bond. Alternatively or additionally, the genes may be linked to the same or equivalent control sequences, so that both genes become expressed within the individual in response to the same stimuli. A wide variety of different control sequences active in different host cells under different conditions are known in the art. These control sequences, including constitutive control sequences, inducible control sequences, and repressible control sequences, can be used, though inducible or repressible sequences are often preferred for applications in which additional control over the timing of gene expression is desired.

It is appreciated by those of skill in the art that administration of cytokine and/or antigen may optionally be combined with the administration of any other desired immune system modulatory factor such as, for example, an adjuvant or other immunomodulatory compound. In some examples, the T_{SCM} cells of the disclosure are administered to a patient in autologous or allogeneic adoptive transfer therapy who is also being treated with an anti-PD-1 therapy, for example, an anti-PD-1 antibody. Anti-PD-1 antibodies and the use thereof are known in the art, for example, as described in Hamid et al. (2013) New England J. Med. 369: 2. Thus, provided are methods of treating a patient comprising the steps of administering T_{SCM} cells and administering an anti-PD-1 antibody.

Dendritic cells can be transfected with nucleic acids (including RNA encoding antigens) by methods known in the art, which include but are not limited to calcium phosphate precipitation, microinjection, or electroporation. The nucleic acids can be added alone or in combination with a suitable carrier, *e.g.,* a pharmaceutically acceptable carrier such as phosphate buffered saline. Alternatively or additionally, the nucleic acid can be incorporated into an expression or insertion vector for incorporation into the cells. Vectors that contain both a promoter and a cloning site into which a polynucleotide can be operatively linked are known in the art. Such vectors are capable of transcribing RNA *in vitro* or *in vivo,* and are commercially available from sources such as Stratagene (La Jolla, CA) and Promega Biotech (Madison, WI). In order to optimize expression and/or *in vitro* transcription, it may be necessary to remove, add, or alter 5' and/or 3' untranslated portions to eliminate extra, potentially inappropriate alternative translation initiation codons or other sequences that may interfere with or reduce expression, either at the level of transcription or translation. Alternatively, consensus ribosome binding sites can be inserted immediately 5' of the start codon to enhance expression. Examples of vectors are viruses, such as baculovirus and retrovirus, bacteriophage, adenovirus, adeno-associated virus, cosmid, plasmid, fungal vectors and other vehicles typically used in the art which have been described for expression in a variety of eukaryotic and prokaryotic hosts, and may be used for gene therapy as well as for simple protein expression. Any suitable vector or delivery method may be used and can readily be selected by one of skill in the art.

Polynucleotides are inserted into vectors using methods and reagents known in the art, including for example, restriction enzymes, synthetic nucleic acid linkers, and oligonucleotides; other functional sequences can be included in the vector and/or associated with the polynucleotide as needed, including, for example, selectable marker genes, enhancer sequences, promoter sequences, start and stop codons, transcription termination signals, RNA processing signals, origins of replication, multiple cloning sites, and T7 and SP6 RNA promoters for in *vitro* transcription of sense and antisense RNA. Other means and functional sequences are known and available in the art and can also be used.

In some methods of PME-CD40L DC maturation, CD40L is delivered to the cell as mRNA. RNA can be obtained by first inserting a DNA polynucleotide into a suitable host cell or by *in vitro* transcription. If a host cell is used, DNA can be inserted into the cell by any appropriate method, *e.g.,* by the use of an appropriate gene delivery vehicle (*e.g.,* liposome, plasmid or vector) or by electroporation. When the cell replicates and the DNA is transcribed into RNA; the RNA can then be isolated using methods well known to those of skill in the art, for example, as set forth in Sambrook, ed. ((2001) Molecular Cloning: A Laboratory Manual, Third Edition (Cold Spring Harbor Laboratory Press, Cold Spring Harbor, New York). For instance, mRNA can be isolated using various lytic enzymes or chemical solutions according to Sambrook (2001), *supra,* or extracted by nucleic-acid-binding resins or other commercially-available products following the instructions provided by the manufacturer.

In preferred DC maturation methods the CD40L expression cassette contains a promoter suitable for *in vitro* transcription, such as the T7 promoter or SP6 promoter. Preferably, the *in vitro* transcribed CD40L or CD40 agonist mRNA is optimized for stability and efficiency of translation, such as, for example, as demonstrated by SEQ ID NO:13 of WO2007117682, which represents an optimized CD40L mRNA wherein ATG codons in the 5' untranslated region have been altered to avoid incorrect initiation of translation.

mRNA stability and/or translational efficiency can also be increased by including 3' UTRs and or 5' UTRs in the mRNA. Preferred examples of 3' UTRs include those from human CD40, beta-actin and rotavirus gene 6. Preferred examples of 5' UTRs include those of CD40L and the translational enhancers in the 5' UTRs of Hsp70, VEGF, spleen necrosis virus RU5, and tobacco etch virus. For example, CD40L expression is normally regulated in part by 3' UTR-mediated mRNA instability, and therefore a large portion of the CD40L 3' UTR is not included in the current CD40L mRNA. CD40L is not normally expressed in DCs, but the CD40 Receptor is expressed in DCs and its expression does not seem to be regulated post-transcriptionally, particularly at the level of mRNA stability, so including the CD40 Receptor 3' UTR or an active fragment thereof at the 3' end or region of the CD40L mRNA would give the RNA 3' untranslated sequence similar to naturally occurring CD40 messages without imparting any unwanted regulatory activity. The benefits and disadvantages of other 5' and 3' UTRs are also known in the art, and one of skill in the art is able to select appropriate UTRs for use in a particular cell type or situation.

Polypeptides and proteins are needed to perform methods of DC maturation; many are commercially available, and others can be obtained by chemical synthesis using a commercially available automated peptide synthesizer such as those manufactured by Perkin Elmer/Applied Biosystems, Inc., Model 430A or 431A, Foster City, CA, USA. The synthesized protein or polypeptide can be precipitated and further purified, for example by high performance liquid chromatography (HPLC). Alternatively, the proteins and polypeptides can be obtained by any known method, including any recombinant method.

It is well know to those skilled in the art that modifications can be made to any peptide to provide it with altered properties. Peptides that may be used in the methods of the disclosure include peptides comprising natural and/or unnatural or synthetic amino acids, including D- and L-amino acids, amino acid analogs, and peptidomimetics.

Some methods require the use of antibodies. Such antibodies can be monoclonal or polyclonal. They can be antibody derivatives or antibody variants, such as, for example, linear antibodies. They can be chimeric, humanized, or totally human. Using a protein or a polypeptide one of skill in the art can generate additionally antibodies which specifically bind to the receptor. A functional fragment or derivative of an antibody also can be used including Fab, Fab', Fab2, Fab'2, and single chain variable regions. Antibodies can be produced in cell culture, in phage, or in various animals, including but not limited to cows, rabbits, goats, mice, rats, hamsters, guinea pigs, sheep, dogs, cats, monkeys, chimpanzees, apes, *etc.* So long as the fragment or derivative retains specificity of binding for the protein or fragment thereof it can be used. Antibodies can be tested for specificity of binding by comparing binding to appropriate antigen to binding to irrelevant antigen or antigen mixture under a given set of conditions. If the antibody binds to the appropriate antigen at least 2, 5, 7, and preferably 10 times more than to irrelevant antigen or antigen mixture then it is considered to be specific. Techniques for making such partially to fully human antibodies are known in the art and any such techniques can be used.

Various methods are known for quantifying the expression of a gene of interest (*e.g.,* CD40L and/or IL-12p35) and include but are not limited to hybridization assays (Northern blot analysis) and PCR-based hybridization assays. In assaying for an alteration in mRNA level such as IL-12 p35 mRNA or CD40L mRNA, the nucleic acid contained in a sample can be first extracted using methods known in the art; commercial kits are available. The mRNA contained in the extracted nucleic acid sample can then be detected by hybridization (*e.g.,* Northern blot analysis) and/or amplification procedures using nucleic acid probes and/or primers, respectively, according to standard procedures known to those of ordinary skill in the art.

Nucleic acid molecules having at least 10 nucleotides and exhibiting sequence complementarity or homology to the nucleic acid to be detected can be used as hybridization probes or primers in diagnostic methods. It is known in the art that a "perfectly matched" probe is not needed for a specific hybridization. Minor changes in probe sequence achieved by substitution, deletion or insertion of a small number of bases do not affect the hybridization specificity. In general, as much as 20% base-pair mismatch (when optimally aligned) can be tolerated. For example, a probe useful for detecting CD40L mRNA is at least about 80% identical to the homologous region of comparable size contained in a previously identified sequence, *e.g.,* see SEQ ID NOS: 1 or 3 of WO2007117682. Alternatively, the probe is at least 85% or even at least 90% identical to the corresponding gene sequence after alignment of the homologous region. The total size of the fragment, as well as the size of the complementary stretches, will depend on the intended use or application of the particular nucleic acid segment. Smaller fragments of the gene will generally find use in hybridization examples, wherein the length of the complementary region may be varied, such as between about 10 and about 100 nucleotides, or even full length according to the complementary sequences of interest for detection.

Nucleotide probes having sequences complementary to a target sequence of a nucleotide over stretches greater than about 10 nucleotides in length will increase stability and selectivity of the hybrid between the probe and the target nucleotide, thereby improving the specificity of particular hybrid molecules obtained. One of skill in the art can design nucleic acid molecules having complementary stretches of more than about 25 and even more preferably more than about 50 nucleotides in length, or even longer where desired. Such fragments may be readily prepared by, for example, directly synthesizing the fragment by chemical means, by application of nucleic acid reproduction technology, such as the PCRTM technology with two priming oligonucleotides (for example, as described in U.S. Patent No. 4,603,102) or by introducing selected sequences into recombinant vectors for recombinant production.

In certain examples, it will be advantageous to employ nucleic acid sequences of the present disclosure in combination with an appropriate means, such as a label, for detecting hybridization and therefore complementary sequences. A wide variety of appropriate indicator means are known in the art, including fluorescent, radioactive, enzymatic or other ligands, such as avidin/biotin, which are capable of giving a detectable signal. A fluorescent label or an enzyme tag, such as urease, alkaline phosphatase, or peroxidase can also be used. In the case of enzyme tags, colorimetric indicator substrates are knownin the art which can be employed to provide a means visible to the human eye or spectrophotometrically, to identify specific hybridization with complementary nucleic acid-containing samples.

As is known in the art, hybridization reactions can be performed under conditions of different "stringency." Relevant conditions include temperature, ionic strength, time of incubation, the presence of additional solutes in the reaction mixture such as formamide, and the washing procedure. Higher stringency conditions are those conditions such as higher temperature and lower sodium ion concentration, which require higher minimum complementarity between hybridizing elements for a stable hybridization complex to form. Conditions that increase the stringency of a hybridization reaction are widely known in the art. One of skill in the art can also utilize, detect, and quantify the amount or level of expression of mRNA using quantitative PCR or high throughput analysis such as Serial Analysis of Gene Expression (SAGE) as described in Velculescu et al. (1995) Science 270:484-487. Other techniques are also known in the art.

General procedures for PCR are known in the art and taught in MacPherson et al. ((2006) PCR: The Basics, Second Edition (Taylor & Francis Group, New York, NY)). However, PCR conditions used for each application reaction are empirically determined. A number of parameters influence the success of a reaction, including annealing temperature and time, extension time, Mg²⁺ ATP concentration, pH, and the relative concentration of primers, templates, and deoxyribonucleotides; adjustment of these parameters to achieve the desired result is known to those of skill in the art.

After amplification, the resulting DNA fragments can be detected by agarose gel electrophoresis followed by visualization with ethidium bromide staining and ultraviolet illumination. A specific amplification of differentially expressed genes of interest can be verified by demonstrating that the amplified DNA fragment has the predicted size, exhibits the predicated restriction digestion pattern, and/or hybridizes to the correct cloned DNA sequence. Other methods for detecting gene expression are known to those skilled in the art. See, for example, International PCT Application No. WO 97/10365, U.S. Pat. Nos. 5,405,783, 5,412,087 and 5,445,934, 5,405,783; 5,412,087; 5,445,934; 5,578,832; and 5,631,734.

A variety of techniques are available in the art for protein analysis and include, but are not limited to radioimmunoassays, ELISA (enzyme linked immunoradiometric assays), "sandwich" immunoassays, immunoradiometric assays, *in situ* immunoassays (using *e.g.,* colloidal gold, enzyme or radioisotope labels), western blot analysis, immunoprecipitation assays, immunofluorescent assays and PAGE-SDS.

Suitably, an effective amount of a cytokine and/or co-stimulatory molecule is delivered to the cells or patient, *in vitro* or *in vivo.* These agents can be delivered as polypeptides, proteins or alternatively, as the polynucleotides or genes encoding them. Cytokines, co-stimulatory molecules and chemokines can be provided as impure preparations (*e.g.,* isolates of cells expressing a cytokine gene, either endogenous or exogenous to the cell) or in a "purified" form. Purified preparations are preferably at least about 90% pure, or alternatively, at least about 95% pure, or yet further, at least about 99% pure. Alternatively, genes encoding the cytokines or inducing agents may be provided, so that gene expression results in cytokine or inducing agent production either in the individual being treated or in another expression system (*e.g.,* an *in vitro* transcription/translation system or a host cell) from which expressed cytokine or inducing agent can be obtained for administration to the individual.

The immunogenicity of the T cells produced by the methods described herein can be determined by well known methodologies, including but not limited to the following:
⁵¹Cr-release lysis assay. Lysis of peptide-pulsed ⁵¹Cr-labeled targets by antigen-specific T cells can be compared. "More active" compositions will show greater lysis of targets as a function of time. The kinetics of lysis as well as overall target lysis at a fixed timepoint (e.g., 4 hours) may be used to evaluate performance, for example, as discussed in Ware et al. (1983) J. Immunol. 131: 1312.
Cytokine-release assay. Analysis of the types and quantities of cytokines secreted by T cells upon contacting modified APCs can be a measure of functional activity. Cytokines can be measured by ELISA or ELISPOT assays to determine the rate and total amount of cytokine production, for example, as discussed in Fujihashi et al. (1993) J. Immunol. Meth. 160:181; Tanquay and Killion (1994) Lymphokine Cytokine Res. 13: 259.
*In vitro* T cell education. T cells can be tested for lytic activity, cytokine-release, polyclonality, and cross-reactivity to the antigenic epitope, for example, as discussed in Parkhurst et al. (1996) Immunol. 157: 2539.
Transgenic animal models. Immunogenicity can be assessed *in vivo* by vaccinating HLA transgenic mice with the compositions of the disclosure and determining the nature and magnitude of the induced immune response. Alternatively, the hu-PBL-SCID mouse model allows reconstitution of a human immune system in a mouse by adoptive transfer of human PBL. These animals may be vaccinated with the compositions and analyzed for immune response as previously mentioned in Shirai et al. (1995) J. Immunol. 154:2733; Mosier et al. (1993) Proc. Natl. Acad. Sci. USA 90:2443.
Proliferation Assays. T cells will proliferate in response to reactive compositions. Proliferation can be monitored quantitatively by measuring, for example, ³H-thymidine uptake, for example, as discussed in Caruso et al. (1997) Cytometry 27:71.
Primate models. A non-human primate (chimpanzee) model system can be utilized to monitor in *vivo* immunogenicities of HLA-restricted ligands. It has been demonstrated that chimpanzees share overlapping MHC-ligand specificities with human MHC molecules thus allowing one to test HLA-restricted ligands for relative *in vivo* immunogenicity, *e.g.,* as discussed in Bertoni et al. (1998) Immunol. 161: 4447.
Monitoring TCR Signal Transduction Events. Several intracellular signal transduction events *(e.g.,* phosphorylation) are associated with successful TCR engagement by MHC-ligand complexes. The qualitative and quantitative analysis of these events have been correlated with the relative abilities of compositions to activate effector cells through TCR engagement, *e.g.,* as discussed in Salazar et al. (2000) Tnt. J. Cancer 85:829; Isakov et al. (1995) J. Exp. Med. 181:375).

As used in the specification and claims, the singular form "a," "an" and "the" include plural references unless the context clearly dictates otherwise. For example, the term "a cell" includes a plurality of cells, including mixtures thereof.

As used herein, the term "comprising" is intended to mean that the compositions and methods include the recited elements, but not excluding others. "Consisting essentially of' when used to define compositions and methods, shall mean excluding other elements of any essential significance to the combination. Thus, a composition consisting essentially of the elements as defined herein would not exclude trace contaminants from the isolation and purification method and pharmaceutically acceptable carriers, such as phosphate buffered saline, preservatives, and the like. Polypeptides or protein that "consist essentially of' a given amino acid sequence are defined herein to contain no more than three, preferably no more than two, and most preferably no more than one additional amino acids at the amino and/or carboxy terminus of the protein or polypeptide. Nucleic acids or polynucleotides that "consist essentially of' a given nucleic acid sequence are defined herein to contain no more than ten, preferably no more than six, more preferably no more than three, and most preferably no more than one additional nucleotide at the 5' or 3' terminus of the nucleic acid sequence. "Consisting of" shall mean excluding more than trace elements of other ingredients and substantial method steps for administering the compositions of this disclosure. Examples defined by each of these transition terms are within the scope of this disclosure.

All numerical designations, *e.g.,* pH, temperature, time, concentration, and molecular weight, including ranges, are approximations which are varied *(*+*)* or *(*-*)* by increments of 0.1. It is to be understood, although not always explicitly stated, that the reagents described herein are merely exemplary and that equivalents of such are known in the art.

The term "antigen" is well understood in the art and includes any substance which is immunogenic, *i.e.,* an immunogen. It will be appreciated that the use of any antigen is envisioned for use in the present disclosure and thus includes but is not limited to a self-antigen (whether normal or disease-related), an antigen of an infectious agent (*e.g.,* a microbial antigen, viral antigen, *etc*.), or some other foreign antigen (*e.g.,* a food component, pollen, *etc*.). The term "antigen" or "immunogen" applies to collections of more than one immunogen, so that immune responses to multiple immunogens may be modulated simultaneously. Moreover, the term includes any of a variety of different formulations of immunogen or antigen.

A "native" or "natural" or "wild-type" antigen is a polypeptide, protein or a fragment thereof which contains an epitope, which has been isolated from a natural biological source, and which can specifically bind to an antigen receptor when presented in a subject as an MHC/peptide complex, in particular a T cell antigen receptor (TCR).

The term "tumor associated antigen," "tumor antigen," or "TAA" refers to an antigen that is associated with a tumor. Examples of well-known TAAs include gp100, MART and MAGE. Other tumor antigens may be specific to a particular tumor in a particular patient.

The terms "major histocompatibility complex" or "MHC" refers to a complex of genes encoding cell-surface molecules that are required for antigen presentation to T cells and for rapid graft rejection. In humans, the MHC is also known as the "human leukocyte antigen" or "HLA" complex. The proteins encoded by the MHC are known as "MHC molecules" and are classified into Class I and Class II MHC molecules. Class I MHC molecules are expressed by nearly all nucleated cells and have been shown to function in antigen presentation to CD8⁺ T cells. Class I molecules include HLA-A, B, and C in humans. Class II MHC molecules are known to function in CD4⁺ T cells and, in humans, include HLA-DP, -DQ, and -DR.

The term "antigen presenting cells (APCs)" refers to a class of cells capable of presenting one or more antigens in the form of peptide-MHC complex recognizable by specific effector cells of the immune system, and thereby inducing an effective cellular immune response against the antigen or antigens being presented. APCs can be intact whole cells such as macrophages, B-cells, endothelial cells, activated T-cells, and dendritic cells; or other molecules, naturally occurring or synthetic, such as purified MHC Class I molecules complexed to β2-microglobulin. While many types of cells may be capable of presenting antigens on their cell surface for T-cell recognition, only dendritic cells have the capacity to present antigens in an efficient amount to activate naive T-cells for cytotoxic T-lymphocyte (CTL) responses.

The term "dendritic cells" (herein also referred to as "DCs") refers to a diverse population of morphologically similar cell types found in a variety of lymphoid and non-lymphoid tissues (see, *e.g.,* Steinman (1991) Ann. Rev. Immunol. 9:271-296). Dendritic cells constitute the most potent and preferred APCs in the organism. DCs can be differentiated from monocytes but are phenotypically distinct from monocytes; for example, CD 14 antigen is not found in dendritic cells but is possessed by monocytes. Also, mature dendritic cells are not phagocytic, whereas monocytes are strongly phagocytosing cells. It has been shown that mature DCs can provide all the signals necessary for T cell activation and proliferation.

The term "immune effector cells" refers to cells capable of binding an antigen and which mediate an immune response. These cells include, but are not limited to, T cells, B cells, monocytes, macrophages, NK cells and cytotoxic T lymphocytes (CTLs), for example CTL lines, CTL clones, and CTLs from tumor, inflammatory, or other infiltrates.

A "naive" immune effector cell is an immune effector cell that has never been exposed to an antigen capable of activating that cell. Activation of naive immune effector cells requires both recognition of the peptide:MHC complex and the simultaneous delivery of a costimulatory signal by a professional APC in order to proliferate and differentiate into antigen-specific armed effector T cells.

As used herein, the term "educated, antigen-specific immune effector cell", is an immune effector cell as defined above which has previously encountered an antigen. In contrast to its naive counterpart, activation of an educated, antigen-specific immune effector cell does not require a costimulatory signal; recognition of the peptide:MHC complex is sufficient.

"Immune response" broadly refers to the antigen-specific responses of lymphocytes to foreign substances. Any substance that can elicit an immune response is said to be "immunogenic" and is referred to as an "immunogen". An immune response of this disclosure can be humoral (via antibody activity) or cell-mediated (via T cell activation).

"Activated", when used in reference to a T cell, implies that the cell is no longer in Go phase, and begins to produce one or more of cytotoxins, cytokines and other related membrane-associated proteins characteristic of the cell type (*e.g.,* CD8⁺ or CD4⁺), and is capable of recognizing and binding any target cell that displays the particular peptide/MHC complex on its surface, and releasing its effector molecules.

As used herein, the phrase "inducing an immune response in a subject" or to induce an immune response in a subject is understood in the art and refers to an increase of at least about 2-fold, or alternatively at least about 5-fold, or alternatively at least about 10-fold, or alternatively at least about 100-fold, or alternatively at least about 500-fold, or alternatively at least about 1000-fold or more in an immune response to an antigen which can be detected or measured, after introducing the antigen into the subject, relative to the immune response (if any) before introduction of the antigen into the subject. In some examples, a treatment is considered to have induced an immune response to an antigen in a subject if an immune response is increased by at least 20%, 30%, 40%, 50%, 60%, 70%, 80%, 90%, 100%, or more in comparison to the immune response exhibited by the subject to the antigen before the treatment. An immune response to an antigen includes but is not limited to the production of an antigen-specific antibody or an increase in the production of antigen-specific antibodies; an increase or decrease in the amount or frequency of an identifiable immune cell type; and the production of an immune cell expressing on its surface a molecule which specifically binds to an antigen. Methods of determining whether an immune response to a given antigen has been induced are well known in the art. For example, antigen-specific antibody can be detected using any of a variety of immunoassays known in the art, including, but not limited to, ELISA, wherein, for example, binding of an antibody in a sample to an immobilized antigen is detected with a detectably-labeled second antibody (*e.g.,* enzyme-labeled mouse anti-human Ig antibody). Increases in immune cell types in response to a treatment are shown in the working examples described herein, such as those discussed in Example 5.

As used herein, the term "cytokine" refers to any one of the numerous factors that exert a variety of effects on cells, for example, inducing growth or proliferation. Nonlimiting examples of cytokines which may be used alone or in combination in the practice of the present disclosure include interleukin-2 (IL-2), stem cell factor (SCF), interleukin-3 (IL-3), interleukin-6 (IL-6), interleukin-12 (IL-12), G-CSF, granulocyte macrophage-colony stimulating factor (GM-CSF), interleukin-1 alpha (IL-1α), interleukin-1L (IL-1L), MIP-11, leukemia inhibitory factor (LIF), c-kit ligand, thrombopoietin (TPO), IL-15, and 1L-17. One example of the present disclosure includes culture conditions in which an effective amount of IL-1β and/or IL-6 is excluded from the medium. Cytokines are readily commercially available, and may be 'natural' purified cytokines or may be recombinantly produced.

The terms "polynucleotide," "nucleic acid," and "nucleic acid molecule" are used interchangeably to refer to polymeric forms of nucleotides of any length. The polynucleotides may contain deoxyribonucleotides, ribonucleotides, and/or their analogs. The term "polynucleotide" includes, for example, a gene or gene fragment, plasmids, vectors, isolated DNA of any sequence, isolated RNA of any sequence, nucleic acid probes, and primers. In addition to a native nucleic acid molecule, a nucleic acid molecule of the present disclosure may also comprise modified nucleic acid molecules. As used herein, mRNA refers to an RNA that can be translated in a dendritic cell. Such mRNAs typically are capped and have a ribosome binding site (Kozak sequence) and a translational initiation codon. As used herein, an RNA corresponding to a cDNA sequence refers to an RNA sequence having the same sequence as the cDNA sequence, except that the nucleotides are ribonucleotides instead of deoxyribonucleotides, as thymine (T) base in DNA is replaced by uracil (U) base in RNA.

The term "peptide" is used in its broadest sense to refer to a compound of two or more subunit amino acids, amino acid analogs, or peptidomimetics. The subunits may be linked by peptide bonds. In another example, the subunit may be linked by other bonds, *e.g.,* ester, ether, *etc.* As used herein the term "amino acid" refers to either natural and/or unnatural or synthetic amino acids, including glycine and both the D and L optical isomers, amino acid analogs and peptidomimetics. A peptide of three or more amino acids is commonly called an oligopeptide if the peptide chain is relatively short, whereas if the peptide chain is long, the peptide is commonly called a polypeptide or a protein.

A "conservative alteration" to a polypeptide or protein is one that results in an alternative amino acid of similar charge density, hydrophilicity or hydrophobicity, size, and/or configuration (*e.g.,* Val for Ile). In comparison, a "nonconservative alteration" is one that results in an alternative amino acid of differing charge density, hydrophilicity or hydrophobicity, size and/or configuration (*e.g.,* Val for Phe). The means of making such modifications are well-known in the art.

The term "genetically modified" means containing and/or expressing a foreign gene or nucleic acid sequence which in turn modifies the genotype or phenotype of the cell or its progeny. In other words, it refers to any addition, deletion or disruption to a cell's endogenous nucleotides.

As used herein, "expression" refers to the processes by which polynucleotides are transcribed into mRNA and mRNA is translated into peptides, polypeptides, or proteins. If the polynucleotide is derived from genomic DNA of an appropriate eukaryotic host, expression may include splicing of the mRNA. Regulatory elements required for expression are known in the art and include promoter sequences to bind RNA polymerase and transcription initiation sequences for ribosome binding. Appropriate vectors for bacterial and/or eukaryotic expression are known in the art and are available commercially.

"Under transcriptional control" is a term understood in the art and indicates that transcription of a polynucleotide sequence (usually a DNA sequence) depends on its being operatively linked to an element which contributes to the initiation of, or promotes, transcription. "Operatively linked" refers to a juxtaposition wherein the elements are in an arrangement allowing them to function.

A "gene delivery vehicle" is defined as any molecule that can carry inserted polynucleotides into a host cell. Examples of gene delivery vehicles include liposomes, biocompatible polymers, including natural polymers and synthetic polymers; lipoproteins; polypeptides; polysaccharides; lipopolysaccharides; artificial viral envelopes; metal particles; and bacteria, or viruses, such as baculovirus, adenovirus and retrovirus, bacteriophage, cosmid, plasmid, fungal vectors and other recombination vehicles typically used in the art which have been described for expression in a variety of eukaryotic and prokaryotic hosts, and may be used for gene therapy as well as for simple protein expression.

"Gene delivery," "gene transfer," "transfection" and the like as used herein, are terms referring to the introduction of an exogenous polynucleotide into a host cell, regardless of the method used for the introduction. Transfection refers to delivery of any nucleic acid to the interior of a cell and may include a variety of techniques such as: electroporation; protein-based, lipid-based and cationic-ion-based nucleic acid delivery complexes; viral vectors; "gene gun" delivery; and various other techniques known in the art. The introduced polynucleotide can be stably maintained in the host cell or may be transiently expressed. In preferred examples, an mRNA is introduced into a DC and is transiently expressed. Stable maintenance typically requires that the introduced polynucleotide either contains an origin of replication compatible with the host cell or integrates into a replicon of the host cell such as an extrachromosomal replicon (*e.g.,* a plasmid) or a nuclear or mitochondrial chromosome. A number of vectors are capable of mediating transfer of genes to mammalian cells and are known in the art.

The sequence of a polynucleotide or portion thereof (or a polypeptide or portion thereof) has a certain percentage of "sequence identity" to another sequence (for example, 80%, 85%, 90%, or 95%) when that percentage of bases or amino acids are the same when the two sequences are aligned and compared. The proper alignment and the percent sequence identity between two sequences can be determined using one of the well-known and publicly available BLAST alignment programs with default parameters.

The term "isolated" means separated from constituents, cellular and otherwise, with which the polynucleotide, peptide, polypeptide, protein, antibody, or fragments thereof, are normally associated with in nature. For example, with respect to a polynucleotide, an isolated polynucleotide is one that is separated from the 5' and 3' sequences with which it is normally associated in the chromosome. A mammalian cell, such as dendritic cell is isolated from an organism if it is removed from the anatomical site from which it is found in an organism. In addition, a "concentrated", "separated" or "diluted" polynucleotide, peptide, polypeptide, protein, antibody, or fragment(s) thereof, is distinguishable from its naturally occurring counterpart in that the concentration or number of molecules per volume is greater than "concentrated" or less than "separated" than that of its naturally occurring counterpart.

"Host cell," "target cell" or "recipient cell" are intended to include any individual cell or cell culture which can be or have been recipients for vectors or the incorporation of exogenous nucleic acid molecules, polynucleotides and/or proteins. It also is intended to include progeny of a single cell. In some instances, a progeny cell may not be completely identical (in morphology or in genomic or total DNA complement) to the original parent cell due to natural, accidental, or deliberate mutation. The cells may be prokaryotic or eukaryotic, and include but are not limited to bacterial cells, yeast cells, animal cells, and mammalian cells, e.g., murine, rat, simian or human.

A "subject" or "patient" is a mammal; in many examples, a patient is a human patient. A subject or patient can also be any other mammal, including a monkey or ape, or any domestic animal such as a dog, cat, horse, *etc.*

By "cancer" is meant the abnormal presence of cells which exhibit relatively autonomous growth, so that a cancer cell exhibits an aberrant growth phenotype characterized by a significant loss of cell proliferation control (*i.e.,* it is neoplastic). Cancerous cells can be benign or malignant. In various examples, cancer affects cells of the bladder, blood, brain, breast, colon, digestive tract, lung, ovaries, pancreas, prostate gland, or skin. The definition of a cancer cell, as used herein, includes not only a primary cancer cell, but also any cell derived from a cancer cell ancestor, including metastasized cancer cells, *in vitro* cultures, and cell lines derived from cancer cells. Cancer includes, but is not limited to, solid tumors, liquid tumors, hematologic malignancies, renal cell cancer, melanoma, breast cancer, prostate cancer, testicular cancer, bladder cancer, ovarian cancer, cervical cancer, stomach cancer, esophageal cancer, pancreatic cancer, lung cancer, neuroblastoma, glioblastoma, retinoblastoma, leukemias, myelomas, lymphomas, hepatoma, adenomas, sarcomas, carcinomas, blastomas, *etc.* When referring to a type of cancer that normally manifests as a solid tumor, a "clinically detectable" tumor is one that is detectable on the basis of tumor mass; *e.g.,* by such procedures as CAT scan, magnetic resonance imaging (MRI), X-ray, ultrasound or palpation. Biochemical or immunologic findings alone may be insufficient to meet this definition.

The term "culturing" refers to the *in vitro* maintenance, differentiation, and/or propagation of cells or in suitable media.

By "enriched" is meant a composition comprising cells present in a greater percentage of total cells than is found in another composition, such as, for example, the tissues where they are present in an organism or a group or mixture of cells in which they were previously present. For example, in the enriched cultures and preparations of T_{SCM} cells made by the methods of the invention, T_{SCM} cells are present in a higher percentage of total cells as compared to their percentage in an *in vitro* culture in which they were produced or cultured. T_{SCM} cells that are in an 'enriched' composition are present as more than 10%, 20%, 30%, 40%, 50%, 60%, or 70% of the cells in that composition. Similarly, by "purified" or "isolated" is intended that a cell type (*e.g.,* T_{SCM} cells) are present as more than 30%, 40%, 50%, 60%, 70%, 80%, 90%, or 95% or 99% of the cells in that composition. Conversely, by "depleted" is intended that the frequency of that cell type is decreased in a particular composition or group of cells, *e.g.,* by at least 10%, 20%, 30%, 40%, 50%, 60%, 70%, 80%, or 90% or more, or 100%. By "enriching" or "enrichment" as used herein is intended that cells are "enriched" using positive selection to selectively or preferentially remove them from a population or group of cells, or that cells are "enriched" using negative selection to selectively or preferentially remove other cells from a starting population or group of cells so that the desired cell type(s) remain behind. Positive and/or negative selection can be readily accomplished using materials and techniques known in the art. For example, cells expressing a particular cell surface marker can be separated from other cells using monoclonal antibodies that bind to the marker and are coupled to columns or magnetic beads; the separation is readily performed according to standard techniques and/or manufacturer or provider directions.

By "cell surface marker" (sometimes herein referred to as "marker" or "cell marker") is intended a molecule expressed on the surface of a cell that can be detected, for example, using labeled antibodies or other means known in the art. A cell surface marker can comprise a protein, glycoprotein, or group of proteins and/or glycoproteins. In some instances a cell surface marker is known to correlate with or be indicative of a particular cell type or one or more cell functions. Certain cell populations can be identified by expression of a particular set or combination of markers, or some subset thereof.

By "positive expression" or "positive for" with reference to a cell surface marker as used herein is generally intended that the marker is expressed at detectable levels on a cell or in a group of cells or population of cells. In some instances, "positive expression" or "positive for" is used to refer to cells that express a particular cell surface marker at levels significantly above background levels or "negative" levels, which can be evaluated by comparison to other cells or other groups or populations of cells, or can be a selected level of expression identified as background or negative. One of skill in the art is familiar with techniques for detecting expression of a marker and for determining the level or levels of expression that distinguish "positive" expression from background or negative expression. Cells that have "positive expression" or are "positive for" a particular marker can exhibit expression that is at least 10%, 20%, 30%, 40%, 50%, 60%, 70%, 100%, 150%, 200%, 300%, 400%, or 500% higher than background expression, or at least 10-fold, 20-fold, 30-fold, 40-fold, 50-fold, 60-fold, or 100-fold or higher than background or "negative" expression. In some examples, the marker is expressed intracellularly but the expression is detectable using techniques known in the art. Generally, expression of a marker is detected with moieties that bind the marker (*e.g.,* antibodies) that are coupled to a fluorescent label or other label that can be measured using a FACS device according to the manufacturers or provider's directions, for example, as demonstrated by the experiments described in the working examples herein.

A "pharmaceutical composition" is intended to include the combination of an active agent with a carrier, inert or active, making the composition suitable for diagnostic or therapeutic use *in vitro* or *in vivo.* The term "pharmaceutically acceptable carrier" encompasses any of the standard pharmaceutical carriers, such as a phosphate buffered saline solution, water, and emulsions, such as an oil/water or water/oil emulsion, and various types of wetting agents. The compositions also can include stabilizers and preservatives. For examples of carriers, stabilizers and adjuvants, see Martin, Remington's Pharmaceutical Sciences, 18th Ed. (Mack Publ. Co., Easton (1990)).

An "effective amount" is an amount sufficient to produce any beneficial or desired results, such as enhanced immune response, treatment, prevention or amelioration of a medical condition (disease, infection, *etc*). An effective amount can be administered in one or more administrations, applications or dosages. Suitable dosages will vary depending on body weight, age, health, disease or condition to be treated and route of administration; methods of determining an effective amount are known in the art. In some examples, an "effective amount" of AGS-004 treatments is the number of AGS-004 doses needed for a particular patient to exhibit a desired result such as a decrease in HIV viral load following ARTI (also referred to as ATI or STI), or to increase the frequency of T_{SCM}s in the patient's blood (for example, as described elsewhere herein), or to exhibit a delayed time to HIV viral rebound following ARTI, or to meet criteria to remain without resuming ART following ARTI. These criteria can vary, but in some instances a patient must be restarted on ART if there are two consecutive tests showing that the patient's CD4+ T cell cound is below 350 cells/mL. In other instances, in addition to the CD4+ T cell count criteria, a patient must also meet viral load criteria of less than 10,000 copies/mL or be restarted on ART. It is understood by those of skill in the art that any positive immune response can provide a benefit to a patient (*e.g.,* an HIV patient or a cancer patient), even if the patient is not completely cured of the HIV infection or cancer, for example, by strengthening the patient's immune response so that other treatments may be more effective than they would have been otherwise.

As used herein, "signaling" means contacting an immature or mature dendritic cell with an IFN-γ receptor agonist, a TNF-α receptor agonist, a CD40L polypeptide or other CD40 agonist. In one example, such agonists are provided externally, (*e.g.,* in the cell culture medium). In another example, the polypeptide agonist is provided via transfection of an immature or mature dendritic cell with a nucleic acid encoding the polypeptide. Alternatively, a nucleic acid aptamer agonist could be provided in the medium or by transfection. In cases where the polypeptide(s) is provided by transfecting a dendritic cell with a nucleic acid encoding the polypeptide, signaling is effected upon translation of an mRNA encoding the polypeptide, rather than upon transfection with the nucleic acid. As used herein, the term "mature dendritic cells" means dendritic cells that demonstrate elevated cell surface expression of co-stimulator molecule CD83, compared to immature DCs (iDCs).

As used herein, by the term "significant difference" is intended that an increase or decrease in a measured parameter is statistically significant as determined using an appropriate statistical test. Such methods are known in the art and the proper test is readily selected by one of skill in the art.

Throughout this disclosure, various publications, patents and published patent specifications are referenced by an identifying citation. The disclosures of these publications, patents and published patent specifications are hereby specifically incorporated by reference into the present disclosure to more fully describe the state of the art to which this disclosure pertains.

The practice of the present disclosure employs, unless otherwise indicated, conventional techniques of molecular biology (including recombinant techniques), microbiology, cell biology, biochemistry and immunology, which are within the skill of the art. Such techniques are known in the art and explained in the literature. In accordance with the above description, the following examples are intended to illustrate, but not limit, the various aspects of this invention.

### EXPERIMENTAL EXAMPLES

### Example 1 - PME-CD40L DC maturation process and evaluation

PME-CD40L DCs were prepared essentially as described in Calderhead et al. ((2008) J. Immunother. 31: 731-41). Briefly, CD40L was cloned from activated T cells that had been stimulated with phorbol 12-myristate 13-acetate (PMA); RT-PCR was performed on total RNA from the T cells using gene-specific CD40L primers to amplify and clone CD40L. Human PBMCs were isolated from leukapheresis collections from healthy volunteers by Ficoll-histopaque density centrifugation. PBMCs were resuspended in culture medium and allowed to adhere to plastic flasks; nonadherent cells were removed and remaining cells were cultured in medium supplemented with GM-CSF (1000 U/ml) and IL-4 (1000 U/ml) for 5-6 days at 37°C, 5% CO₂. DCs were harvested, washed in PBS, re-suspended in chilled Viaspan® media (DuPont Pharma®), and placed on ice. DCs were mixed with CD40L mRNA and antigen-encoding mRNA and electroporated. Immediately after electroporation, DCs were washed and re-suspended in medium that was supplemented with GM-CSF and IL-4. DCs were cultured for either 4 or 24 hours at 37°C in low-adherence plates with additional maturation stimuli as described below.

Immature DCs were phenotypically matured on day 5 of culture by adding TNF-α, IFN-γ, and PGE₂. On day 6, DCs were harvested and electroporated with antigen and CD40L mRNA as described above, and cultured in media containing GM-CSF and IL-4 for 4 hrs prior to harvest or formulation for vaccine production.

For flow cytometric analysis, DCs were harvested and re-suspended in chilled PBS/ 1% FCS, then mixed with phycoerythrin (PE) or FITC-conjugated antibodies specific for CD1a, CD209, human leukocyte antigen (HLA)-ABC, HLA-DR, CD80, CD86, CD38, CD40, CD25, CD123, CD83, CCR6, CCR7, CD70, and CD14; isotype-matched antibodies were used as controls. After thorough washing, fluorescence analysis was performed with a FACScalibur flow cytometer (BD Biosciences™) and CellQuest software (BD Biosciences™). Chemotaxis of DCs was measured by migration through a 8-µm pore size polycarbonate filter. IL-10 and IL-12 in the DC supernatants were determined using ELISA.

For CTL induction, mature DCs electroporated with mRNAs were co-cultured with CD8+ purified T-cells. For the first three days the cells were cultured in media supplemented with IL-2 and IL-7; on day 4, the media was supplemented with IL-2. On day 7, the CD8⁺ cells were harvested and restimulated with DC in media supplemented with IL-2 and IL-7. CTL assays were performed 3 days after the second or third stimulation.

### Example 2 - Use of PME-CD40L DCs to Induce T_{SCM} CTLs

PME-CD40L DCs expressing the MART-1 tumor antigen were used to expand a population of T_{SCM} CTLs *in vitro.* PME-CD40L DCs were produced and transfected with mRNA encoding the MART-1 antigen and cocultured with PBMCs isolated from the same patient. Data shown in Figure 1 illustrates the ability of these PME-CD40L DCs to prime and/or expand a population of T_{SCM} CTLs and also demonstrates that T_{SCM} CTLs can be identified in co-cultures of T-cells and PME-CD40L DCs. T_{SCM} CTLs were identified as shown in Figure 1 by multi-color flow cytometry as cells that are phenotypically CD8+/CD95+/CD28+/CCR7+/CD45RA+. Further testing of these cells revealed that a proportion of the MART-1+ T_{SCM} CTLs were multi-functional, with 1.8% expressing TNF-α, 3.2% expressing CD107a, and 1.5% expressing IFN-γ.

### Example 3 -T_{SCM} cells as markers of immune response in HIV subjects

As part of a clinical trial (diagrammed schematically in Figure 3), human HIV patients were treated with PME-CD40L DCs encoding HIV antigens ("AGS-004") prepared as in WO2006042177 (Healey et al.); WO2007117682 (Tcherepanova et al.); DeBenedette et al. (2008) J. Immunol. 181: 5296-5305; Calderhead et al. (2008) J. Immunother. 31: 731-4; and WO 2006031870 (Nicolette et al.)*.* In this clinical trial, the PME-CD40L DCs contained "GNVR", the RNA antigen payload encoding the antigens GAG (G), Nef (N), VPR (V), and Rev (R). Patients were treated with multiple doses of AGS-004 and the anti-HIV immune response was determined post therapy by monitoring viral load and immune response.

Longitudinal blood draws were collected prior to AGS-004 dosing at visit 2 or visit 3, after two doses (visit 6) and four doses (visit 8) of AGS-004 administered during anti-retroviral therapy ("ART"), and at two time points during Structured Treatment Interruption ("STI"), at visit 9 and visit 13. Visit 13 blood draws were collected after two additional doses of AGS-004 during STI.

PBMCs were collected from the subjects and stimulated *in vitro* with autologous PME-CD40L DCs encoding HIV antigens from the subject. Multi-color flow cytometry was then used to determine the proliferation capacity and phenotype of stimulated HIV-specific CD4 and CD8 T cells and to identify the activation state of HIV-specific T cells. T cell proliferation was measured by CFSE dye dilution in combination with multi-color flow cytometry. In this manner, CFSE was a proliferation marker, while CD27, CD8, CD28, CCR7, CD3, CD45RA, and CD4 were phenotypic markers (see Figure 4). Absolute numbers of AGS-004 responding T cells (cells/ml) were determined using Trucount tubes. Patients exhibiting proliferating Tscm cells (see Figure 5) were selected for comparison of this response with viral load analysis.

Patients were evaluated for viral load and immune response to determine the relationship between time to viral rebound after anti-retroviral therapy (ART) interruption and the anti-GNVR T cell response. Patients on ART have no detectable HIV viral load, but when the drug therapy is withdrawn the virus will rebound. A delay in viral rebound and/or a reduced amount of viral load following ARTI in a patient is indicative of an anti-HIV immune response in that patient.

"Viral rebound" is defined here as the time to detectable virus in the plasma of a patient as measured by standard assays such as, for example, the Roche COBAS® AMPLICOR® HIV-1 MONITOR Test (v1.5, which has a sensitivity of 50 HIV RNA copies/mL) and the Abbott RealTime HIV-1 assay (which has a sensitivity of 40 HIV RNA copies/mL). A delay in viral rebound occurs when the time to detectable virus is longer in a patient compared to other patients and/or compared to the expected time of viral rebound in the absence of effective treatment. Typically, in the absence of any effective anti-HIV treatment, viral rebound would occur within two weeks of ART interruption.

For the patients for whom data is shown in Figure 2, the days to detectable viral load (detectable virus in the plasma) is shown in Table 1 below:

**Table 1: Days to Detectable Viral Load**

| Subject | Days |
|---|---|
| 021-002 | 26 |
| 023-001 | 14 |
| 025-004 | 15 |
| 011-009 | 60 |
| 023-002 | 155 |
| 032-001 | 43 |
| 011-005 | 27 |

For some patients, additional criteria distinguish a desired clinical outcome. For example, patient 011-005 had almost the same days to detectable viral load as patient 021-002; however, patient 011-005 is considered a good "controller" of HIV because their viral load was much lower and after 12 weeks of ART interruption the patient only had 1690 HIV RNA copies/mL and was able to continue without ART for six additional months. In contrast, patient 021-002 had a viral load that rebounded to much higher levels (never below 54,600 copies/mL) and had to resume ART after the 12 week assessment period (see, *e.g.,* Figure 6).

The frequency of CFSElo T cells in a patient represents the percentage of T cells proliferating *in vitro* after restimulation with the DCs that were transfected with RNA encoding the viral antigens GNVR. Single time point data for the frequency of CFSElo T cells was analyzed for each patient at visit 9 and visit 13 post AGS-004 treatment, but neither time point data showed an association between the percentage of proliferating T cells and extended time to viral rebound in the patient. However, analysis of the cumulative percentage of proliferating T cells measure at both visit 9 (after 4 doses AGS-004) and visit 13 (after 2 additional doses AGS-004) revealed a positive association between increases in the percentage of proliferating T cells displaying the expression of the markers CD27⁺/CD28⁺/CD45RA⁺ and a delay in viral rebound and/or decreased viral load.

This proliferating T cell phenotype (*i.e.,* expressing CD27, CD28, and CD45RA) describes T_{SCM} cells for both the CD4 and CD8 T cell populations. As shown in Figure 2A, B, C, and D, four patients with proliferating CD27⁺/CD28⁺/CD45RA⁺ CD4 T_{SCM} cells (Figure 2A) and CD27⁺/CD28⁺/CD45RA⁺ CD8 T_{SCM} cells (Figure 2C) displayed a longer time to viral rebound and/or decreased viral load after ART interruption (ATI) than three other patients who showed a rapid time to viral rebound. These four patients exhibiting an extended time to viral rebound and/or decreased viral load also showed a marked difference in the percent change of proliferating CD27⁺/CD28⁺/CD45RA⁺ CD4 T_{SCM} cells (Figure 2B) and CD27⁺/CD28⁺/CD45RA⁺ CD8 T_{SCM} cells (Figure 2D) measured from baseline (visit 3) and after administration of AGS-004 determined as the mean response measured by averaging the response detected at visit 9 and visit 13. The percent change from visit 3 was determined by dividing the mean average percentage of cells in the CD4 (Figure 2B) or CD8 (Figure 2D) CD27⁺/CD28⁺/CD45RA⁺ T cell gate determined at visit 9 and visit 13 by the percent of cells in the CD4 or CD8 CD27⁺/CD28⁺/CD45RA⁺T cell determined at visit 3 (baseline).

As shown by the experiments discussed above for which data is also shown, for example, in Figure 2 and Figures 4 through 11, immune responses to AGS-004 could be detected in both CD4 and CD8 T_{SCM} cell subsets prior to and during interruption of Anti-Retroviral Therapy (ART). A proportion of these subjects exhibited an extended time to viral rebound after AGS-004 administration during interruption of ART.

These experiments indicate that AGS-004-induced T_{SCM} cells play a role in the induction of an anti-HIV response in patients that can delay viral rebound during interruption of ART and serve as an indicator of a patient's immune response. Also, patients having cells with greater expression of PD-1, a marker associated with dysfunctional CD4- and CD8-T cell activation, are more likely to exhibit shorter times to viral rebound. Thus, decreased PD-1 expression can be an indicator of a successful immune response to HIV. These experiments indicate that AGS-004 immunotherapeutic intervention may reverse the induction of T cell exhaustion, leading to control of viral replication by inducing long-term immunity.

### Example 4 -Association of PD-1 cell phenotypes with immune response in chronically infected HIV patients

HIV infection induces immune dysregulation of both CD4 and CD8 T cell compartments. The anti-viral T cell pools in chronically infected patients are characterized by an exhausted activation phenotype, which is unable to control virus replication. ART can suppress HIV replication leading to stabilizing of CD4 T cell numbers and improving immune function. However, latent virus is not eliminated and the residual T cell pools do not overcome this state of T cell exhaustion. During non-controlled viral replication, the expression of Programmed Cell Death-1 (PD-1) on activated T cells is associated with T cell exhaustion and poor viral control. AGS-004 is a personalized dendritic cell (DC) loaded with RNA encoding autologous Gag, Nef, Vpr, and Rev. AGS-004-001 is a Phase 2 trial designed to assess the efficacy and safety of AGS-004 during a 12-week ART structured treatment interruption (ARTI) in chronic HIV-1 infected subjects. This immunotherapeutic intervention is intended to reverse the induction of T cell exhaustion leading to control of viral replication by inducing long-term immunity.

Longitudinal blood draws were collected prior to AGS-004 dosing, after four doses of AGS-004 administered during ART, and after two doses during ARTI. Phenotype of HIV-specific CD4 and CD8 T cells and their proliferation capacity were determined by multi-color flow cytometry to determine the activation state of HIV specific T cells. Measurements of viral load were performed during these same time-frames. Associations between T cell proliferation, T cell activation state and viral control were determined.

Positive changes in the magnitude of T cell proliferation to viral antigens after AGS-004 administration were detected in subjects with extended time to viral rebound during ARTI. Both activated CD4 and CD8 T cells lacking PD-1 were increased after AGS-004 administration in subjects with an extended time to viral rebound versus subjects with faster time to viral rebound measured during STI. Furthermore, subjects displaying rapid viral rebound had a greater number of exhausted CD57⁺/PD-1⁺ CD4 and CD8 effector T cells compared to subjects with an extended time to viral rebound.

### Example 5 -Immunogenicity of AGS-004 Dendritic Cell Therapy in Patients Treated during Acute HIV Infection

Enhancing HIV-1 specific immunity without CD4 T cell activation may clear productively infected cells, a key aspect of HIV eradication strategies. AGS-004 consists of matured autologous dendritic cells (PME-CD40L DCs) co-electroporated with *in vitro* transcribed RNA encoding HIV proteins Gag, Nef, Rev, and Vpr ("GNVR") amplified from participants' pre-ART plasma. These cells are also referred to herein as GNVR DCs or "AGS-004 DC vaccine."

A clinical trial was conducted with HIV patients who initiated Anti-Retroviral Therapy ("ART") within 45 days of acute HIV infection and had HIV RNA < 50 copies/ml for more than 6 months. In this instance, AHI was defined as negative or indeterminate EIA or a negative HIV RNA test within 40 days of detectable plasma HIV RNA. Monthly doses of AGS-004 were administered on ART and immune responses ("IR") were assessed after 3-4 doses of AGS-004, at week 12 or 16 of the trial. Here, a positive immune response was defined as an increase in the number of CD28+/CD45RA- CD8+ CTL cells that was two-fold or greater compared to the baseline level and also was 3 standard deviations or more above a negative control. Patients showing an increase in immune response after three doses of AGS-004 were eligible for voluntary analytic treatment interruption with continued monthly doses of AGS-004; all patients shown in Table 2 met the criteria for an immune response (IR). ART was restarted if a patient's CD4 count fell below 350 cells/mm³ or showed more than a 20% decline in absolute CD4 count or percentage, or if HIV RNA was confirmed to be at or above 10,000 copies/ml. The frequency of resting CD4+ T-cell infection was measured by quantitative viral outgrowth assays at baseline and after three doses of AGS-004 (at week 10).

PBMCs were collected from patients pre- and post-treatment with AGS-004 and were cultured *in vitro* with autologous AGS-004 DC vaccine. After *in vitro* stimulation, cells were prepared for multi-color flow cytometry and evaluated for expression of surface markers including CD28, CD45RA, CD27, and CCR7 (see Figure 12). HIV antigen-reactive CTL subsets were identified and assessed for function, *e.g.,* for the production of cytokines (*e.g.,* IFN-gamma, TNF-alpha, and IL-2); expression of cytolytic markers (*e.g.*, Granzyme b, CD107); and proliferation (*e.g.*, as shown by BrdU staining).

The demographic and clinical characteristics of these patients are shown in Table 2 below (SCA is Single Copy Assay for HIV; RCI is Resting Cell Infection). Table 3 shows the antigenic response meeting positivity criteria; as indicated in the table, two patients received AGS-004 with only three of Gag, Nef, Vpr, and Rev.

**Table 2: Demographic and Clinical Characteristics of Patients Receiving AGS-004 DC Therapy for AHI**

| Participant ID | Age (years) | Race/ethnicity | Baseline CD4 count (cells/mm²) | Baseline SCA (cps/mL) | Length of ATI (days) | Reason for ART restart | Viral suppression after ATI | Baseline frequency of RCI (IUPM)^{a} | Post-treatment Frequency of RCI (IUPM)^{a} |
|---|---|---|---|---|---|---|---|---|---|
| 51-100 | 34 | African American | 662 | <0.6 | 36 | VL > 10,000 | Yes | 0.266 | 0.140 |
| 51-102 | 31 | African American | 397 | -- | 268+^{b} | N/A | N/A | 0.767 | 0.572 |
| 54-100 | 56 | White, non-hispanic | 574 | <0.4 | 90 | VL > 10,000/ >20% ↓ CD4 | Yes | 0.179 | 0.067 |
| 54-101 | 26 | White, non-hispanic | 482 | <0.5 | 147 | VL > 10,000 | Yes | 0.043 | 0.049 |
| 54-102 | 51 | African American | 937 | <0.5 | 58 | >20% ↓ CD4 | Yes^{c} | 0.088 | 0.195 |
| 54-104 | 26 | African American | 714 | 0.8 | 41 | >20% ↓ CD4 | Yes^{c} | 0.525 | 0.691 |

| | | | | | | | | | |
|---|---|---|---|---|---|---|---|---|---|
| ^{a}IUMP = infectious units per million; Remains on ATI; ^{c} Viremic after initial re-suppression due to non-compliance with daily ART adherence | | | | | | | | | |

**Table 3: Antigenic Response Meeting Positivity Criteria**

| Participant ID | GNVR | GAG | Nef | Vpr | Rev |
|---|---|---|---|---|---|
| 51-100 | + | - | - | - | - |
| 51-102 | + | + | + | + | + |
| 54-100 | + | - | - | - | + |
| 54-101* | GNR+ | - | - | NT | + |
| 54-102 | + | - | - | - | + |
| 54-104* | GVR+ | - | NT | + | - |

| | | | | | |
|---|---|---|---|---|---|
| * Two participants received AGS-004 with 3 gene products; NT = not tested | | | | | |

In this trial, all participants met the criteria for positive immune response and ATI (Anti-retroviral Treatment Interruption). The median duration of ATI was 58 days, with a range of 36 to 147 days, and one patient remained in ATI after 268 days. The baseline SCA (Single Copy Assay for HIV) was less than 1c/ml in all patients. Only one patient (54-100) had a greater than 2-fold decrease in frequency of RCI at Week 10 but maintained ATI for 90 days.

Figures 13 through 18 show multi-functional immune responses ("MIFs") and viral load trajectories for the patients before, during, and after ATI with AGS-004. The figures show absolute numbers of CD28+/CD45RA- CTLs for each marker as well as viral load trajectories. Antigen-specific response for each MIF was determined by subtracting the absolute number of CTLs in the control GFP response plus 3 times the SD from GNVR antigen responses at Week 0 and Week 12. An asterisk above a bar in the bar graph indicates a CTL response that met criteria for positivity, defined in these experiments as at least a 2-fold increase in the absolute number of CTLs for a given (test) antigen determined post-dosing in comparison to the number of CTLs for said antigen per-dosing (*i.e*., at Week 0).

This clinical trial demonstrated that AGS-004 DC therapy was safe, well-tolerated, and led to increased HIV-specific immune responses. The one participant with a greater than two-fold decrease in the frequency of RCI at week 10 underwent ATI for 90 days. These results indicate that DC therapy could deplete persistent HIV infection in ART-suppressed patients following administration of anti-latency therapy.

## Claims

1. An in vitro method for obtaining an autologous population of cells enriched for T_{SCM} cells suitable for introduction into a human patient comprising the steps of:
a) preparing PME-CD40L mature DCs from a tissue source isolated from a human patient;
b) culturing PBMCs obtained from said patient *in vitro* with said PME-CD40L mature DCs in a coculture for a time sufficient to induce an increase in the number of T_{SCM} cells in said coculture;
c) enriching said T_{SCM} cells from said coculture.

2. The method of claim 1, wherein said PME-CD40L mature DCs are loaded with an antigen.

3. The method of claim 2, wherein said DCs are loaded with said antigen by transfection with RNA encoding said antigen.

4. The method of claim 3, wherein said RNA is prepared from cells of an HIV-infected patient.

5. The method of claim 3, wherein said antigen is prepared from cancer cells of a cancer patient.

6. The method of claim 5, wherein said T_{SCM} cells are CD8+, CD95+, CD28+, CCR7+, and CD45RA+.

7. The method of claim 1, wherein said T_{SCM} cells are CD27⁺, CD28⁺, and CD45RA⁺.

8. The method of claim 7, wherein said T_{SCM} cells are enriched as cells positive for CD27, CD28, and CD45RA.

9. The method of claim 1, further comprising combining said T_{SCM} cells with a pharmaceutically acceptable carrier.

## Patentansprüche

1. Ein *in vitro* Verfahren zum Erhalten einer autologen Population von Zellen, die mit T_{SCM} - Zellen angereichert ist, die für die Einführung dieser in einen menschlichen Patienten geeignet sind, umfassend die Schritte von:
a) zubereiten von PME-CD40L reifen DCs aus einer Gewebequelle, die aus einem menschlichen Patienten isoliert wurde:
b) kultivieren der PMBCs, die von jenem Patienten erhalten wurde *in vitro* mit jenen PME-CD40L reifen DCs in einer Co-Kultur für eine Zeit, die ausreichend ist, um eine Erhöhung der T_{SCM}-Zellen-Anzahl in jener Co-Kultur zu induzieren;
c) anreichern jener T_{SCM}-Zellen aus jener Co-Kultur.

2. Das Verfahren gemäß Anspruch 1, wobei jene PME-CD40L reife DCs mit einem Antigen beladen werden.

3. Das Verfahren gemäß Anspruch 2, wobei jene DCs mit jenem Antigen durch Transfektion mit RNA, die für jenes Antigen kodiert, beladen werden.

4. Das Verfahren gemäß Anspruch 3, wobei jene RNA aus Zellen eines HIV-infizierten Patienten präpariert wird.

5. Das Verfahren gemäß Anspruch 3, wobei jenes Antigen aus Krebszellen eines Krebspatienten präpariert wird.

6. Das Verfahren gemäß Anspruch 5, wobei jene T_{SCM}-Zellen CD8+, CD95+, CD28+, CCR7+ und CD45RA+ sind.

7. Das Verfahren gemäß Anspruch 1, wobei jene T_{SCM}-Zellen CD27+, CD28+ und CD45RA+ sind.

8. Das Verfahren gemäß Anspruch 7, wobei jene T_{SCM}-Zellen als Zellen, die positiv für CD27, CD28 und CD45RA, angereichert sind.

9. Das Verfahren gemäß Anspruch 1, ferner umfassend das Kombinieren jener T_{SCM}-Zellen mit einem pharmazeutischen annehmbaren Träger.

## Revendications

1. Procédé in vitro d'obtention d'une population autologue de cellules enrichies en cellules T_{SCM} aptes à être introduites chez un patient humain comprenant les étapes consistant à :
a) préparer des cellules dendritiques PME-CD40L matures à partir d'une source tissulaire isolée d'un patient humain ;
b) cultiver les PBMC obtenues à partir dudit patient *in vitro* avec lesdites CD PME-CD40L matures dans une coculture pendant une durée suffisante pour induire une augmentation du nombre de cellules T_{SCM} dans ladite coculture ;
c) enrichir lesdites cellules T_{SCM} à partir de ladite coculture.

2. Procédé selon la revendication 1, dans lequel lesdites CD PME-CD40L matures sont chargées avec un antigène.

3. Procédé selon la revendication 2, dans lequel lesdites CD sont chargées avec ledit antigène par transfection avec un ARN codant pour ledit antigène.

4. Procédé selon la revendication 3, dans lequel ledit ARN est préparé à partir de cellules d'un patient infecté par le VIH.

5. Procédé selon la revendication 3, dans lequel ledit antigène est préparé à partir de cellules cancéreuses d'un patient atteint de cancer.

6. Procédé selon la revendication 5, dans lequel lesdites cellules T_{SCM} sont CD8+, CD95+, CD28+, CCR7+ et CD45RA+.

7. Procédé selon la revendication 1, dans lequel lesdites cellules T_{SCM} sont CD27⁺, CD28⁺ et CD45RA⁺.

8. Procédé selon la revendication 7, dans lequel lesdites cellules T_{SCM} sont enrichies en tant que cellules positives pour CD27, CD28 et CD45RA.

9. Procédé selon la revendication 1, comprenant en outre combiner lesdites cellules T_{SCM} avec un support pharmaceutiquement acceptable.
